(19) Europäisches Patentamt
European Patent Office
Office européen des brevets

(11) **EP 3 859 431 B1**

(12) **EUROPEAN PATENT SPECIFICATION**

(45) Date of publication and mention of the grant of the patent:
**16.04.2025 Bulletin 2025/16**

(21) Application number: **19881048.3**

(22) Date of filing: **13.09.2019**

(51) International Patent Classification (IPC):
***G02C 7/04*** *(2006.01)* ***A61L 15/22*** *(2006.01)*
***A61L 15/24*** *(2006.01)* ***A61L 15/26*** *(2006.01)*
***A61L 27/16*** *(2006.01)* ***A61L 27/18*** *(2006.01)*
***A61L 27/26*** *(2006.01)* ***A61L 29/04*** *(2006.01)*
***A61L 29/06*** *(2006.01)* ***A61L 29/08*** *(2006.01)*
***A61L 31/04*** *(2006.01)* ***A61L 31/06*** *(2006.01)*
***A61L 31/10*** *(2006.01)*

(52) Cooperative Patent Classification (CPC):
(C-Sets available)
**A61L 29/14; A61L 15/225; A61L 15/50; A61L 27/34; A61L 27/50; A61L 29/085; A61L 31/10; A61L 31/14;** A61L 2400/10; A61L 2420/02; A61L 2420/08 (Cont.)

(86) International application number:
**PCT/JP2019/036066**

(87) International publication number:
**WO 2020/095539 (14.05.2020 Gazette 2020/20)**

(54) **MEDICAL DEVICE AND METHOD FOR MANUFACTURING SAME**

MEDIZINISCHE VORRICHTUNG UND VERFAHREN ZUR HERSTELLUNG DAVON

DISPOSITIF MÉDICAL ET SON PROCÉDÉ DE FABRICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.11.2018 JP 2018211150**

(43) Date of publication of application:
**04.08.2021 Bulletin 2021/31**

(73) Proprietor: **Toray Industries, Inc.**
**Tokyo 103-8666 (JP)**

(72) Inventors:
- **IIMORI, Hirokazu**
**Okazaki-shi, Aichi 444-8522 (JP)**
- **KITAGAWA, Rumiko**
**Otsu-shi, Shiga 520-8558 (JP)**
- **KATO, Tomohiro**
**Otsu-shi, Shiga 520-8558 (JP)**
- **NAKAMURA, Masataka**
**Otsu-shi, Shiga 520-8558 (JP)**

(74) Representative: **Prüfer & Partner mbB**
**Patentanwälte · Rechtsanwälte**
**Sohnckestraße 12**
**81479 München (DE)**

(56) References cited:
**EP-A1- 2 746 835**
**WO-A1-2013/024880**
**WO-A1-2017/169873**
**WO-A1-2018/207586**
**JP-A- 2015 206 011**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61L 15/225, C08L 33/08;**
**A61L 15/225, C08L 33/10;**
**A61L 15/225, C08L 39/06;**
**A61L 27/34, C08L 33/08;**
**A61L 27/34, C08L 33/10;**
**A61L 27/34, C08L 39/06;**
**A61L 29/085, C08L 33/08;**
**A61L 29/085, C08L 33/10;**
**A61L 29/085, C08L 39/06;**
**A61L 31/10, C08L 33/08;**
**A61L 31/10, C08L 33/10;**
**A61L 31/10, C08L 39/06**

## Description

[Technical Field]

The present invention relates to a medical device and a method for producing the same.

[Background Art]

Since materials used in a medical device may come into contact with a living body, there is a situation where they are required to be soft. These soft materials are used for introduction into a living body or for coating a surface of a living body. Examples of the medical device using soft materials include contact lenses, cell culture sheets, scaffold materials for tissue regeneration and medical electrodes for skin. Soft materials are also used in cosmetic devices such as facial packs.

When the medical device is brought into contact with the mucous membrane in a living body or worn on eyes, it is important that properties of the material surface, such as surface wettability and lubricity, are improved, namely, surface modification is performed, in order to improve the biocompatibility.

The surface modification of the medical device includes those in which at least two types of acidic polymers are allowed to exist in a polymer layer on the surface of a substrate as mentioned in Patent Literature 1.

The method of modifying the surface includes a method called an LbL method (Layer by Layer method) in which a surface layer of each of two polymer materials having opposite charges is formed one by one, as mentioned in Patent Literature 2. There are also a method in which a polymer layer is formed by noncovalently bonding different materials (Patent Literature 1), a method in which two types of hydrophilic polymers are crosslinked on the surface of a medical device by a heat treatment (Patent Literature 3) and the like.

WO 2017/169873 A1 discloses a medical device comprising a substrate and a polymer layer, wherein the polymer layer is provided on at least a part of a surface of the substrate, the water content of the medical device in hydrated state is in a range of 28% by mass or more and 50% by mass or less, and the substrate has a 2-alkoxyethyl group. The polymer layer contains one type of a copolymer having an amide structure.

[Citation List]

[Patent Literature]

[Patent Literature 1] JP 2017-23374 A
[Patent Literature 2] JP 2005-538767 W
[Patent Literature 3] JP 2014-533381 W

[Summary of Invention]

[Technical Problem]

However, although the modified surface obtained by the above method can achieve satisfactory surface wettability and lubricity in the initial stage of water wetting, there was still a problem in maintaining the above surface properties when used for a long time in the actual use environment. For example, when the medical device having the modified surface is an ophthalmic lens, it is conceivable that the medical device will be used for a long time under dry conditions in winter or under air conditioning in summer as the assumed use environment. In this case, in order to prevent or reduce eye dryness, it is considered to be necessary for the ophthalmic lens to maintain its surface wettability for a long time. However, in the currently used ophthalmic lens, there was still a problem in maintaining the surface wettability for a long time. Since the modified surface layer of the ophthalmic lens requires to be a very thin layer with respect to the substrate, it is difficult to maintain the surface wettability for a long time as compared with other medical devices which are physically capable of retaining water in a surface layer by thickening the surface layer.

In the medical electrode for skin, it is important that the surface wettability of the electrode can be maintained for a long time in order to maintain the contact resistance with the skin.

The present invention has been made in view of aforementioned problems of prior art. Thus, it is an object of the present invention to provide a medical device which is capable of maintaining the surface wettability for a long time.

[Solution to Problem]

To achieve the above object, the present invention has the following structures.
The present invention is directed to a medical device comprising a substrate and a polymer layer, wherein the polymer layer is provided on at least a part of a surface of the substrate and satisfies the following requirements:

(a) a water content of the medical device in hydrated state is in a range of 28% by mass or more and 50% by mass or less;
(b) the substrate has a 2-alkoxyethyl group; and
(c) the polymer layer contains one type of a carboxylic acid group-containing polymer and one type of a copolymer having an amide structure.

[Advantageous Effects of Invention]

According to the present invention, it is possible to obtain a medical device capable of maintaining surface wettability for a long time.

[Brief Description of the Drawings]

FIG. 1 is a top view of an aluminum pedestal in which a lens-shaped medical device is disposed.
FIG. 2 is a side view of an aluminum pedestal in which a lens-shaped medical device is disposed.
FIG. 3 is an example of the measurement results of a wet value (Fluidity factor) by multi speckle-diffusing wave spectroscopy (hereinafter referred to as MS-DWS method).

[Description of Embodiments]

The present invention has the following structures. The present invention is directed to a medical device comprising a substrate and a polymer layer, wherein the polymer layer is provided on at least a part of a surface of the substrate and satisfies the following requirements:

(a) a water content of the medical device in hydrated state is in a range of 28% by mass or more and 50% by mass or less;
(b) the substrate has a 2-alkoxyethyl group; and
(c) the polymer layer contains one type of a carboxylic acid group-containing polymer and one type of a copolymer having an amide structure.

Hereinafter, the polymer layer provided on the surface of the substrate may be simply referred to as surface polymer layer.

<Water Content of Medical Device>

In the medical device of the present invention, the water content in hydrated state is in a range of 28% by mass or more and 50% by mass or less.
When the water content in hydrated state of the entire medical device is 28% by mass or more, a water passage from the inside of the substrate to the surface polymer layer is formed, and the water existing inside the substrate is easily supplied to the surface polymer layer even when placed in a dry state. This is preferable because a medical device having a long surface wettability retention time is easily formed.
When the water content in hydrated state of the entire medical device is 50% by mass or less, it is possible to reduce the shrinkage of hydrophilic groups responsible for the hydrous property as a result of occurrence of shape changes associated with hydrogen bonding as it dries. In order to increase the water content in hydrated state of the entire medical device to more than 50% by mass, it is necessary to introduce many hydrophilic groups into the medical device. Hydrophilic groups generate hydrogen bonds in the material as they dry. The increase in hydrogen bonds causes a shape change which causes the medical device to shrink leading to curing of the material.
Here, "hydrated state" used herein means the following states. First, a medical device is immersed in a phosphate buffered saline solution at 25°C for 1 day or more. After taking the medical device out of the phosphate buffered saline solution at 25°C, the medical device is immediately sandwiched between two pieces of water absorbent

gauze (for example, "Haize (registered trademark)" Gauze VP-150: manufactured by OZU CORPORATION) from above and below, and then water on the surface is removed by lightly pressing. Once again, the medical device is sandwiched between two pieces of water absorbent gauze from above and below and then water on the surface is removed by lightly pressing. The state of the medical device immediately after these operations is defined as "hydrated state". Meanwhile, "dry state" means a state immediately after the medical device is dried in a vacuum dryer at 40°C for 16 hours or more and taken out of the vacuum dryer. Each of various measurement data of "hydrated state" and "dry state", which will be mentioned later, means the data obtained by promptly performing the measurement after completing each of the above-mentioned operations.

<Substrate>

It is possible to use, as the substrate used in the present invention, both a hydrous substrate and a non-hydrous substrate. In the present invention, "hydrous" is defined as a state where the water content is 10% or more in hydrated state, whereas, "non-hydrous" is defined as a state where the water content is less than 10% in hydrated state.

The water content can be calculated, for example, by measuring the mass in hydrated state (W1) and the mass in dry state (W2), followed by calculation based on each of the measurement results by the following equation.

$$\text{Water content (\%)} = 100 \times (W1 - W2)/W1$$

However, in the medical device of the present invention, the water content in hydrated state is in a range of 28% by mass or more and 50% by mass or less, and it is preferable to use a hydrous substrate to realize the water content in this range.

Examples of the hydrous substrate include a hydrogel and a silicone hydrogel. Of these, the silicone hydrogel is preferable because of having excellent flexibility and high oxygen permeability. For example, when the silicone hydrogel is used as the substrate for ophthalmic lens, its flexibility gives excellent comfort and high oxygen permeability enables wearing for a longer time, which is preferable.

<Substrate Having 2-Alkoxyethyl Group>

The substrate used in the present invention has a 2-alkoxyethyl group. Examples of the 2-alkoxyethyl group include a 2-methoxyethyl group, a 2-ethoxyethyl group, a 2-isopropropoxyethyl group and the like. It is considered that the carbon-oxygen bond concentration in the alkoxy moiety is higher and more water as intermediate water can be contained. Of these, in view of the fact that the surface wettability of the thus obtained medical device can be maintained for a long time, and that the followability is excellent in the case of a medical device used in close contact with skin and eyeballs because of low elastic modulus, a 2-methoxyethyl group or a 2-ethoxyethyl group is preferable, and a 2-methoxyethyl group is particularly preferable.

It is more preferable that the substrate used in the present invention has a 2-alkoxyethyl group in a range of 5% by mass or more and 40% by mass or less. The content of the 2-alkoxyethyl group in the substrate is preferably 5% by mass or more, more preferably 7% by mass or more, and still more preferably 10% by mass or more, since the retention time of the surface wettability can be lengthened. Meanwhile, since the substrate does not easily become brittle, the content is preferably 40% by mass or less, more preferably 35% by mass or less, still more preferably 30% by mass or less, and yet more preferably 25% by mass or less. Regarding the upper limit value and the lower limit value in the above-mentioned range, any lower limit value and any upper limit value may be combined.

The 2-alkoxyethyl group can retain intermediate water. The intermediate water means water having an intermediate property between water which is only attached to the surface and crystallization water which does not evaporate. Therefore, it is considered that the water inside the dried substrate (particularly, water held as intermediate water) can be easily supplied to the modified surface polymer layer.

The substrate having a 2-alkoxyethyl group can be obtained, for example, by a method in which a composition for forming a substrate mentioned layer is cured by heat or light.

First, a composition for forming a substrate which is cured by light or heat is injected into a forming mold. The forming mold is made of resin, glass, ceramics, metal, **etc.,** and when photopolymerization is used, a material allowing light of the wavelength used for polymerization to pass through, usually resin or glass, is used. Subsequently, the forming mold filled with the composition for forming a substrate is irradiated with active rays such as ultraviolet rays, visible rays or a combination thereof, or placed in an oven or a liquid tank, followed by heating to cure the composition for forming a substrate. There can also be used a method in which both photocuring and thermal curing are used in combination, for example, thermal curing is performed after photocuring, or conversely, photocuring is performed after thermal curing. In the case of photocuring, it is common to irradiate the composition with light from a light source such as a mercury lamp, a fluorescent lamp, a black light, an LED, X-rays or an electron beam irradiator in a short time (usually 1 hour or less). In the

case of thermal curing, it is preferable to perform under the conditions that the temperature is gradually raised from around room temperature and raised to a high temperature of 60°C to 200°C over several hours to several tens of hours, in order to maintain the uniformity and quality of the obtained cured product and to enhance the reproducibility.

The composition for forming a substrate used in the present invention contains at least a polymerizable compound having a 2-alkoxyethyl group. The composition for forming a substrate may further contain other polymerizable raw materials, a non-polymerizable solvent, a polymerization initiator, a polymerization catalyst and the like.

<Polymerizable Compound Having 2-Alkoxyethyl Group>

It is possible to use, as a polymerizable compound having a 2-alkoxyethyl group, 2-methoxyethyl (meth)acrylate, 2-ethoxyethyl (meth)acrylate, 2-(2-ethoxyethoxy)ethyl (meth)acrylate, diethylene glycol monoethyl ether (meth)acrylate, 2-[2-(2-methoxyethoxy)ethoxy]ethyl (meth)acrylate, 2-isopropoxyethyl (meth)acrylate, 2-methoxyethyl (meth)acrylamide, methyl[2-(vinyloxy)ethyl] ether and the like.

Here, the term (meta) as used herein means any methyl substitution. Therefore, for example, the term "(meth) acrylate" means both methacrylate and acrylate. The same applies to the terms such as "(meth)acrylamide" and "(meth) acryloyl".

Of the polymerizable compounds, 2-methoxyethyl (meth)acrylate, 2-ethoxyethyl (meth)acrylate and 2-methoxyethyl (meth)acrylamide are preferable, and 2-methoxyethyl(meth)acrylamide is particularly preferable, in view of the fact that, in the process for forming a substrate, the formation of the substrate can be completed in a short time because of high curing rate.

Examples of other polymerizable raw materials, which may be contained in the composition for forming a substrate, include the followings.

<Other Polymerizable Raw Materials: Hydrophilic Polymerizable Compound>

It is preferable to use a hydrophilic polymerizable compound as other polymerizable raw materials, since it become easier to set the water content in hydrated state of the obtained medical device at 28% by mass or more and 50% by mass or less. The hydrophilic polymerizable compound is preferably contained in the composition for forming a substrate in a range of 5% by mass or more and 50% by mass or less. If the content of the hydrophilic polymerizable compound is 5% by mass or more, the obtained medical device achieves sufficient water content and high water wettability. If the content of the hydrophilic polymerizable compound is 50% by mass or less, it is possible to reduce deterioration of the oxygen permeability of the obtained medical device, occurrence of the cloudiness due to lack of compatibility with other polymerizable raw materials, or poor gel strength due to excessive swelling. The lower limit of the content of the hydrophilic polymerizable compound is preferably 5% by mass, more preferably 8% by mass, still more preferably 10% by mass, and yet more preferably 12% by mass. The upper limit of the content is preferably 50% by mass, more preferably 45% by mass, still more preferably 40% by mass, and yet more preferably 35% by mass. Any combination of the upper limit value and the lower limit value may be used.

Examples of such hydrophilic polymerizable compound include low molecular compounds having a hydrophilic radically polymerizable group, such as methacrylic acid, acrylic acid, itaconic acid, 2-hydroxyethyl methacrylate, 2-hydroxyethyl acrylate, 2-hydroxypropyl methacrylate, 2-hydroxypropyl acrylate, glycerol methacrylate, polyethylene glycol methacrylate, N,N-dimethylacrylamide, N-methylacrylamide, dimethylaminoethyl methacrylate, methylenebisacrylamide, diacetone acrylamide, N-vinylpyrrolidone, N-vinylcaprolactam, N-vinylacetamide and N-vinyl-N-methylacetamide.

<Other Polymerizable Raw Materials: Linear Polymerizable Silicone Compound>

The substrate used in the medical device of the present invention preferably include a unit derived from a linear polymerizable silicone compound represented by the general formula (1) for the following reasons.

By using a composition for forming a substrate, which containing a polymerizable silicone compound, a medical device having high oxygen permeability and excellent elongation can be obtained. Meanwhile, because of the hydrophobicity of silicone, when a hydrophilic polymerizable compound is contained in the composition for forming a substrate as other polymerizable raw materials, it is difficult to mix with them, and as a result, some polymerizable silicone compound may remain on the substrate as an unreacted product. Such unreacted residue is not preferable in consideration of biological safety since there is a risk of elution in the use environment.

Therefore, it is preferable to use a linear polymerizable silicone compound having a polymerizable group at the terminal. Since the polymerizable silicone compound is linear, the polymerization reaction easily proceeds as compared with the polymerizable silicone compound taking the form of a bulk which is not linear, thus making it possible to suppress that the unreacted product remains on the substrate. When the above-mentioned hydrophilic polymerizable compound is

contained in the composition for forming a substrate, the molecular weight of the linear silicone moiety is adjusted to be small in a range in which desired properties can be obtained, thus making it possible to facilitate mixing with the above-mentioned hydrophilic polymerizable compound.

[Chemical Formula 1]

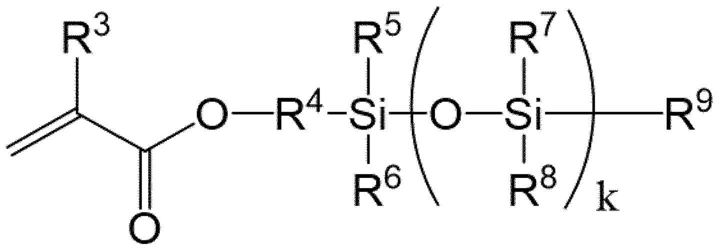

・・(1)

In the general formula (1), $R^3$ represents a hydrogen atom or a methyl group. $R^4$ represents a divalent organic group having 1 to 20 carbon atoms. $R^5$ to $R^8$ each independently represent an alkyl group having 1 to 20 carbon atoms or an aryl group having 6 to 20 carbon atoms. $R^9$ is an optionally substituted alkyl group having 1 to 20 carbon atoms or an optionally substituted aryl group having 6 to 20 carbon atoms. k represents an integer in a range of 1 to 200.

In the general formula (1), $R^3$ represents a hydrogen atom or a methyl group. Of these, a methyl group is more preferable from the viewpoint of easy availability.

In the general formula (1), $R^4$ represents a divalent organic group having 1 to 20 carbon atoms. Examples thereof include alkylene groups such as a methylene group, an ethylene group, a propylene group, a butylene group, a pentylene group, an octylene group, a decylene group, a dodecylene group and an octadecylene group; and arylene groups such as a phenylene group and a naphthylene group. These alkylene groups and arylene groups may be either linear or branched. If the number of carbon atoms of $R^4$ is 20 or less, it is possible to suppress the difficulty in obtaining compatibility with the hydrophilic monomer, and if the number of carbon atoms is 1 or more, there will be a decrease in fear of tearing due to reduced elongation of the obtained medical device. The number of carbon atoms of $R^4$ is more preferably 1 to 12, and particularly preferably 2 to 8. Regarding $R^4$, an alkylene unit may be substituted with an oxygen atom or a sulfur atom, and the hydrogen atom adjacent to carbon may be substituted with a hydroxyl group, an amino group or a halogen atom. Examples of suitable substituent when $R^4$ is substituted include substituents such as a hydroxyl group, a carboxyl group, a sulfonic acid group, a phosphoric acid group, an ester, an ether, an amide, and a combination thereof. Of these, a substituent selected from a hydroxyl group, an ester, an ether and an amide is preferable in that decomposition of the silicone moiety does not easily occur, and a hydroxyl group or an ether is more preferable in that the transparency of the obtained medical device is enhanced.

More preferable examples of $R^4$ include an ethylene group, a propylene group, a butylene group, and a divalent organic group represented by the following formulas (a1) to (a4).

$-CH_2CH(OH)CH_2-$ (a1)

$-CH_2CH(OH)CH_2OCH_2CH_2CH_2-$ (a2)

$-CH_2CH_20CH_2CH_2CH_2-$ (a3)

$-CH_2CH_2OCH_2CH_2OCH_2CH_2CH_2-$ (a4)

Of these, a propylene group and a divalent organic group represented by formulas (a1) to (a4) are preferable, and a propylene group or a divalent organic group represented by formula (a2) is particularly preferable.

In the general formula (1), $R^5$ to $R^8$ each independently represent an optionally substituted alkyl group having 1 or more and 20 or less carbon atoms, or an optionally substituted aryl group having 6 or more and 20 or less carbon atoms. Examples thereof include a methyl group, an ethyl group, an n-propyl group, an isopropyl group, an n-butyl group, an s-butyl group, a t-butyl group, an n-pentyl group, an isopentyl group, an s-pentyl group, a neopentyl group, a hexyl group, a heptyl group, an octyl group, a nonyl group, a decyl group, a dodecyl group, an eicosyl group, a phenyl group, a naphthyl group and the like. These alkyl group and aryl group may be either linear or branched. If the substituent is an optionally substituted alkyl group having 1 to 20 carbon atoms or an optionally substituted aryl group having 6 to 20 carbon atoms, the silicone content is relatively reduced, thus making it possible to suppress deterioration of the oxygen permeability of the obtained medical device. The number of carbon atoms of the alkyl group or the aryl group is more preferably 1 to 12, still more preferably 1 to 6, and yet more preferably 1 to 4. Examples of the substituent when the alkyl group or the aryl group is

substituted include an aldehyde group, a carboxyl group, an alcohol group, an alkoxy group, an ether group, a halogen group, an alkylene glycol group, an alkylthio ether group, an amino group, a nitro group, a cyano group, a sulfuric acid group and a phosphoric acid group.

In the general formula (1), $R^9$ represents an optionally substituted alkyl group having 1 to 20 carbon atoms, or an optionally substituted aryl group having 6 to 20 carbon atoms. When the number of carbon atoms of $R^9$ is 1 or more, it is possible to suppress that the polysiloxane chain is easily hydrolyzed, and when the number of carbon atoms is 20 or less, it is possible to prevent deterioration of the oxygen permeability of the medical device. An alkyl group having 1 to 10 carbon atoms or an aryl group having 6 to 10 carbon atoms is more preferable, an alkyl group having 1 to 6 carbon atoms is still more preferable, and an alkyl group having 1 to 4 carbon atoms is yet more preferable. Preferable examples of the alkyl group having 1 to 20 carbon atoms and the aryl group having 6 to 20 carbon atoms include a methyl group, an ethyl group, an n-propyl group, an isopropyl group, an n-butyl group, an s-butyl group, a t-butyl group, an n-pentyl group, an isopentyl group, an s-pentyl group, a neopentyl group, a hexyl group, a heptyl group, an octyl group, a nonyl group, a decyl group, a dodecyl group, an eicosyl group, a phenyl group, a naphthyl group and the like. These alkyl group and aryl group may be either linear or branched. Examples of the substituent when the alkyl group or the aryl group is substituted include an aldehyde group, a carboxyl group, an alcohol group, an alkoxy group, an ether group, a halogen group, an alkylene glycol group, an alkylthio ether group, an amino group, a nitro group, a cyano group, a sulfuric acid group and a phosphoric acid group.

In the general formula (1), k represents an integer optionally having distribution of 1 or more and 200 or less. When k is 200 or less, it is easy to suppress that the compatibility with the hydrophilic monomer deteriorates because of excessive hydrophobic silicone moieties. When k is 1 or more, oxygen permeability and shape recovery can be obtained. k is more preferably 1 or more and 100 or less, still more preferably 2 or more and 50 or less, and particularly preferably 3 or more and 20 or less. The lower limit of k is preferably 1, more preferably 2, and still more preferably **3.** The upper limit of k is preferably 200, more preferably 100, still more preferably 50, and yet more preferably 20. Any lower limit value and any upper limit value may be used in combination. However, when k has distribution, k is calculated based on the number-average molecular weight of a monofunctional linear silicone monomer and rounded down to the nearest integer. The monofunctional linear silicone monomer used in the composition for forming a substrate may be used alone or in combination of a plurality of types having different **k.** As the monofunctional linear silicone monomer used in the composition for forming a substrate, a plurality of types of monofunctional linear silicone monomers having different chemical structures other than k may be used in combination.

<Polymerization Initiator>

It is possible to use, as the polymerization initiator, a compound which is decomposed or reacts with light (including ultraviolet rays and electron beams) or heat to generate radicals. It is possible to use, as the polymerization catalyst, a catalyst containing, as central metal, ruthenium, copper, iron or nickel which is a living radical polymerization catalyst. In particular, it is preferable to use a ruthenium pentamethylcyclopentadienyl complex and an amino alcohol as a polymerization catalyst in that the polymerization proceeds even in a hydrophilic raw material.

Specific examples of the polymerization initiator include 2-methyl-[4-(methylthio)phenyl]-2-morpholinopropan-1-one, 2-dimethylamino-2-(4-methylbenzyl)-1-(4-morpholin-4-yl-phenyl)-butan-1-one, 2-benzyl-2-dimethylamino-1-(4-morpholinophenyl)-butanone-1,2,4,6-trimethylbenzoylphenylphosphine oxide, bis(2,4,6-trimethylbenzoyl)-phenylphosphine oxide, bis(2,6-dimethoxybenzoyl)-(2,4,4-trimethylpentyl)-phosphine oxide, 1-phenyl-1,2-propanedione-2-(o-ethoxycarbonyl)oxime, 1,2-octandione, 1-[4-(phenylthio)-2-(O-benzoyloxime)], 1-phenyl-1,2-butadione-2-(o-methoxycarbonyl)oxime, 1,3-diphenylpropanetrione-2-(o-ethoxycarbonyl)oxime, ethanone, 1-[9-ethyl-6-(2-methylbenzoyl)-9H-carbazol-3-yl]-,1-(0-acetyloxime), 4,4-bis(dimethylamino)benzophenone, 4,4-bis(diethylamino)benzophenone, ethyl p-dimethylaminobenzoate, 2-ethylhexyl-p-dimethylaminobenzoate, ethyl p-diethylaminobenzoate, diethoxyacetophenone, 2-hydroxy-2-methyl-1-phenylpropan-1-one, benzyl dimethyl ketal, 1-(4-isopropylphenyl)-2-hydroxy-2-methylpropan-1-one, 4-(2-hydroxyethoxy)phenyl-(2-hydroxy-2-propyl)ketone, 1-hydroxycyclohexyl-phenyl ketone, benzoin, benzoin methyl ether, benzoin ethyl ether, benzoin isopropyl ether, benzoin isobutyl ether, benzophenone, methyl o-benzoylbenzoate, 4-phenylbenzophenone, 4,4-dichlorobenzophenone, hydroxybenzophenone, 4-benzoyl-4'-methyldiphenyl sulfide, alkylated benzophenone, 3,3',4,4'-tetra(t-butylperoxycarbonyl)benzophenone, 4-benzoyl-N,N-dimethyl-N-[2-(1-oxo-2-propenyloxy)ethyl]benzenemethanaminium bromide, (4-benzoylbenzyl)trimethylammonium chloride, 2-hydroxy-3-(4-benzoylphenoxy)-N,N,N-trimethyl-1-propenaminium chloride monohydrate, 2-isopropylthioxanthone, 2,4-dimethylthioxanthone, 2,4-diethylthioxanthone, 2,4-dichlorothioxanthone, 2-hydroxy-3-(3,4-dimethyl-9-oxo-9H-thioxanthen-2-yloxy)-N,N,N-trimethyl-1-propanaminium chloride, 2,2'-bis(o-chlorophenyl)-4,5,4',5'-tetraphenyl-1,2-biimidazole, 10-butyl-2-chloroacridone, 2-ethylanthraquinone, benzyl, 9,10-phenanthrenequinone, camphorquinone, diphenyl sulfide derivatives, bis($\eta$5-2,4-cyclopentadien-1-yl)-bis(2,6-difluoro-3-(1H-pyrrol-1-yl)-phenyl) titanium, thioxanthone, 2-methylthioxanthone, 2-chlorothioxanthone, 4-benzoyl-4-methyl phenyl ketone, dibenzyl ketone, fluorenone, 2,3-diethoxyacetophenone, 2,2-dimethoxy-2-phenyl-2-phenylacetophenone, 2-hydroxy-2-methylpropiophenone, p-t-butyldichloroacetophenone, benzyl methoxyethyl acetal, anthraquinone, 2-t-butylanthraquinone, 2-aminoanthraquinone, β-chloranthraquinone, anthrone, benzanthrone, dibenzsuberone, methyleneanthrone, 4-azidobenzalacetophenone, 2,6-bis(p-azidobenzylidene)cyclohexane, 2,6-bis(p-azidobenzylidene)-4-methylcyclohexanone, naphthalenesulfonyl chloride, quinolinesulfonyl chloride, N-phenylthioacridone, benzthiazole disulfide, triphenylphosphine, carbon tetrabromide, tribromophenylsulfone, benzoyl peroxide and eosin; and a combination of photoreducing dyes such as methylene blue and reducing agents such as ascorbic acid and triethanolamine. Two or more of these polymerizable compounds may be contained.

The photopolymerization initiator is preferably an acylphosphine oxide-based polymerization initiator in that the polymerization can be initiated by an inexpensive LED lamp or black light having a maximum wavelength in the vicinity of 300 nm. Specific examples of the acylphosphine oxide compound include 2,4,6-trimethylbenzoylphenylphosphine oxide, bis(2,4,6-trimethylbenzoyl)-phenylphosphine oxide, bis(2,6-dimethoxybenzoyl)-(2,4,4-trimethylpentyl)-phosphine oxide and the like. Meanwhile, since these acylphosphine oxide-based polymerization initiators absorb light in the vicinity of 300 nm, yellowing may occur if a large amount of the polymerization initiator remains on the substrate, and the polymerization initiator has a plurality of benzoyl groups and therefore has high hydrophobicity. Therefore, if the content of the acylphosphine oxide-based polymerization initiator in the composition for forming a substrate is high, precipitation may occur when photopolymerized together with the hydrophilic raw material, leading to formation of a foreign substance. Since the 2-alkoxyethyl group is a non-hydrogen-bonding hydrophilic group, the obtained substrate is a hydrous substrate without secondary cross-linking due to hydrogen bonding. Therefore, if the substrate contains a foreign substance, tearing easily occurs from the foreign substance as a starting point.

The content of the acylphosphine oxide-based polymerization initiator in the composition for forming a substrate is preferably 0.4% by mass or less, and more preferably 0.3% by mass or less, in order to obtain a substrate which does not easily tear after the peeling step or lens formation. The content of the acylphosphine oxide-based polymerization initiator is preferably 0.05% by mass or more, and preferably 0.2% by mass or more, in that the polymerization reaction can be quickly performed without causing phase separation of the hydrophilic and hydrophobic raw materials.

An intramolecular hydrogen abstraction type polymerization initiator is preferable in that it is liquid and is easily mixed with a hydrophilic raw material, and can form a substrate which is hard to tear. Examples of the hydrogen abstraction type polymerization initiator include methyl benzoylformate and "Omnirad" (registered trademark) 754 (manufactured by IGM Group B.V). Meanwhile, the hydrogen abstraction type polymerization initiator is inferior in reaction initiation efficiency when an inexpensive LED lamp or black light having a maximum wavelength in the vicinity of 300 nm is used alone. Therefore, it is preferable that both the acylphosphine oxide-based polymerization initiator and the intramolecular hydrogen abstraction type polymerization initiator are contained in the composition for forming a substrate in that it is possible to simultaneously achieve both excellent initiation efficiency in the polymerization reaction and the property capable of forming a substrate which is difficult to tear and does not cause yellowing.

When the acylphosphine oxide-based polymerization initiator and the intramolecular hydrogen abstraction type polymerization initiator are used in combination, it is preferable that the acylphosphine oxide-based polymerization initiator is contained in a range of 0.05% by mass to 0.4% by mass and the intramolecular hydrogen abstraction type polymerization initiator is contained in a range of 0.2% by mass to 20% by mass. The content of the intramolecular hydrogen abstraction type polymerization initiator in the composition for forming a substrate is preferably 0.2% by mass or more, preferably 0.5% by mass or more, and particularly preferably 1% by mass or more, in that a substrate having a desired shape and properties can be obtained. The content of the intramolecular hydrogen abstraction type polymerization initiator is preferably 20% by mass or less, more preferably 5% by mass or less, and particularly preferably 2% by mass or less, in that a substrate having low yellowness can be obtained without remaining on the substrate.

<Non-Polymerizable Solvent>

The composition for forming a substrate may contain a non-polymerizable solvent. It is possible to apply, as the non-polymerizable solvent, various organic and inorganic solvents. The content of the non-polymerizable solvent is preferably 15 parts by mass or more, more preferably 20 parts by mass or more, and particularly preferably 30 parts or more, based on 100 parts by mass of the total amount of all the polymerizable compounds in that the hydrophilic polymerizable compound and other polymerizable raw materials can be easily dissolved and a homogeneous substrate can be obtained. If a large amount of the non-polymerizable solvent exists in the substrate immediately after curing, voids are easily generated by removing in the subsequent step, so that the amount is preferably 50 parts by mass or less, and particularly preferably 40 parts by mass or less, based on 100 parts by mass of the total amount of all the polymerizable compounds, in that the strength of the obtained substrate can be easily increased.

Examples of the non-polymerizable solvent include water; alcohol-based solvents such as methyl alcohol, ethyl alcohol, normal propyl alcohol, isopropyl alcohol, normal butyl alcohol, isobutyl alcohol, t-butyl alcohol, t-amyl alcohol, tetrahydrolinalol, ethylene glycol, diethylene glycol, triethylene glycol, tetraethylene glycol and polyethylene glycol; glycol ether-based solvents such as methyl cellosolve, ethyl cellosolve, isopropyl cellosolve, butyl cellosolve, propylene glycol

monomethyl ether, diethylene glycol monomethyl ether, triethylene glycol monomethyl ether, polyethylene glycol monomethyl ether, ethylene glycol dimethyl ether, diethylene glycol dimethyl ether, triethylene glycol dimethyl ether and polyethylene glycol dimethyl ether; ester-based solvents such as ethyl acetate, butyl acetate, amyl acetate, ethyl lactate and methyl benzoate; aliphatic hydrocarbon-based solvents such as normal hexane, normal heptane and normal octane; alicyclic hydrocarbon-based solvents such as cyclohexane and ethylcyclohexane; ketone-based solvents such as acetone, methyl ethyl ketone and methyl isobutyl ketone; aromatic hydrocarbon-based solvents such as benzene, toluene and xylene; and petroleum-based solvents. These solvents may be used alone or in combination of two or more types thereof.

<Polymer Layer>

In the medical device of the present invention, a polymer layer is provided on at least a part of the surface of the substrate. The polymer layer contains one type of a carboxylic acid group-containing polymer and one type of a copolymer having an amide structure.

In the medical device of the present invention, "the polymer layer is provided on at least a part of the surface of the substrate" means that the polymer layer may be provided only on a part of the surface of the substrate, or the polymer layer may be provided over the entire surface of the substrate. For example, it is preferable that the polymer layer is provided on the surface of the substrate where skin adhesiveness and surface lubricity are required. For example, in the case of a medical device used in contact with a part of the mucous membrane on the surface of the human body, following cases are assumed: "case of having the polymer layer on the surface of both the side in contact with the mucous membrane and the side not in contact with the mucous membrane (the side in contact with air)" and "case of providing the polymer layer only on the necessary part of the surface on the side not in contact with the mucous membrane (the side in contact with air)".

Examples of the method of forming the polymer layer on at least a part of the surface of the substrate include a method in which the substrate is spray-coated or spin-coated with a polymer solution. Examples of the method of forming the polymer layer only on the necessary part of the surface of the substrate on the side in contact with air include a method of masking the part other than the necessary part during coating. Examples of the method of forming the polymer layer over the entire surface of the substrate include, in addition to the method of spray-coating or spin-coating, a method in which the substrate is put into a polymer solution for forming a polymer layer and heated to form the polymer layer on the surface of the substrate.

In the present invention, the polymer layer is formed as a layer on at least a part of the surface of the substrate, and contains one type of a carboxylic acid group-containing polymer and one type of a copolymer having an amide structure. The polymer layer may contain two or more types of carboxylic acid group-containing polymers, and it is particularly preferable that the polymer layer contains only one type of a carboxylic acid group-containing polymer. The polymer layer may contain a copolymer having two or more types of amide structures, and it is particularly preferable to contain a copolymer having only one type of an amide structure.

The polymer layer may be covalently bonded to the substrate, but does not necessarily have a covalent bond.

In the present invention, "one type of a polymer" means that a polymer synthesized from the same monomer type is counted as one type. If the constituent monomer species are the same, they are counted as the same type of polymer even if they have different molecular weights. Polymers synthesized from different monomer species are counted as different types of polymers.

<Carboxylic Acid Group-Containing Polymer>

The monomer constituting the carboxylic acid group-containing polymer is preferably a monomer having an allyl group, a vinyl group and a (meth) acryloyl group in terms of high polymerizability, and particularly preferably a monomer having a (meth) acryloyl group.

The carboxylic acid group-containing polymer may be either a homopolymer or a copolymer. When the carboxylic acid group-containing polymer is a copolymer, a monomer having no carboxylic acid group may be contained as a copolymerization component. However, a monomer unit having an amide structure is not included. In terms of being capable of having a carboxylic acid group at high density, the carboxylic acid group-containing polymer is preferably composed only of a monomer component having a carboxylic acid group, and more preferably a homopolymer.

Preferable examples of the carboxylic acid group-containing polymer include polymethacrylic acid, polyacrylic acid, polyvinylbenzoic acid, poly(thiophene-3-acetic acid), polymaleic acid, poly(meth)acryloyloxyethyl-succinic acid, poly(meth)acryloyloxyethyl hexahydrophthalic acid, poly(meth)acryloyloxyethyl-phthalic acid, poly(meth)acryloyloxyethyl-2-hydroxyethyl-phthalic acid, polynorbornenecarboxylic acid, poly(meth)acryloyloxyethyl succinate, and salts thereof.

The weight-average molecular weight of the carboxylic acid group-containing polymer is preferably in a range of 10,000 or more and 500,000 or less. The weight-average molecular weight of the carboxylic acid group-containing

polymer is preferably 10,000 or more, more preferably 100,000 or more, and still more preferably 200,000 or more, in that it enables the formation of a surface polymer layer having high lubricity and scrubbing resistance. The weight-average molecular weight of the carboxylic acid group-containing polymer is preferably 500,000 or less, more preferably 400,000 or less, and still more preferably 300,000 or less, in that a uniform surface polymer layer can be formed without becoming a foreign substance. Any lower limit value and any upper limit value may be used in combination.

As the molecular weight, the weight-average molecular weight in terms of polyethylene glycol measured by gel permeation chromatography (aqueous solvent) is used.

<Copolymer Having Amide Structure>

Examples of the copolymer having an amide structure include a copolymer containing a monomer having an amide structure as a monomer component.

The monomer having an amide structure is preferably a monomer selected from a monomer having a (meth) acrylamide structure and N-vinylcarboxylic acid amide (including cyclic ones) in terms of ease of polymerization. Suitable examples of the monomer include N-vinylpyrrolidone, N-vinylcaprolactam, N-vinylacetamide, N-methyl-N-vinylacetamide, N-vinylformamide, N,N-dimethylacrylamide, N,N-diethylacrylamide, N-isopropylacrylamide, N-(2-hydroxyethyl) acrylamide, acryloylmorpholine and acrylamide. Of these, N-vinylpyrrolidone and N,N-dimethylacrylamide are preferable in terms of lubricity, and N,N-dimethylacrylamide is most preferable.

If the copolymer having an amide structure has poor compatibility with the carboxylic acid group-containing polymer, it causes uneven precipitation and cloudiness. Therefore, the copolymerizable monomer other than the monomer having an amide structure contained in the copolymer having an amide structure is preferably a monomer having a group such as a carboxylic acid group, an alcohol group or an ethylene glycol group.

Examples of the carboxylic acid group-containing monomer include methacrylic acid, acrylic acid, itaconic acid and the like. Examples of the alcohol group-containing monomer include 2-hydroxyethyl (meth)acrylate, 2-hydroxypropyl (meth)acrylate, glycerol (meth)acrylate and the like. Examples of the ethylene glycol group-containing copolymerizable monomer include methoxyethyl (meth)acrylate, polyethylene glycol (meth)acrylate and the like.

Of these, a carboxylic acid group-containing monomer having high hydrophilicity and capable of adjusting the hydrophilicity with a small amount is particularly preferable.

Examples of the copolymer having an amide structure include a (meth)acrylic acid/N-vinylpyrrolidone copolymer, a (meth)acrylic acid/N,N-dimethylacrylamide copolymer, a 2-acrylamide-2-methylpropanesulfonic acid/N-vinylpyrrolidone copolymer and a 2-acrylamide-2-methylpropanesulfonic acid/N,N-dimethylacrylamide copolymer. A (meth) acrylic acid/N,N-dimethylacrylamide copolymer is most preferable. A ternary copolymer or a multi-component copolymer in which a copolymerization component is further added to these copolymers is also suitable. In that case, preferable examples of the copolymerization component to be added include hydrophilic (meth)acrylates such as 2-hydroxyethyl (meth)acrylate, 2-methoxyethyl (meth)acrylate, 2-ethoxyethyl (meth)acrylate and 2-isopropoxyethyl (meth)acrylate; and monomers having an amide structure, such as N-vinylpyrrolidone, N-vinylcaprolactam, N-vinylacetamide, N-methyl-N-vinylacetamide, N-vinylformamide, N,N-dimethylacrylamide, N,N-diethylacrylamide, N-isopropylacrylamide, N-(2-hydroxyethyl)acrylamide, acryloylmorpholine and acrylamide.

In terms of simultaneously achieving both high surface lubricity and the property of maintaining surface wettability for a long time, the content ratio of the carboxylic acid group-containing polymer and the copolymer having an amide structure in the polymer layer of the medical device of the present invention, namely, $C_A$ in the following equation (b1) is preferably 0.8 or less, more preferably 0.5 or less, and particularly preferably 0.3 or less.

$$C_A = \text{weight of polymer having an amide structure/weight of polymer layer} \quad \text{(b1)}$$

<Method of Forming Polymer Layer>

Examples of the method of forming a layer containing one type of a carboxylic acid group-containing polymer and one type of a copolymer having an amide structure on the surface of the substrate include a method in which a substrate is immersed in a solution containing a polymer and then heated, a method in which a substrate is heated after coating with a solution containing a polymer, and the like. It is possible to apply, as the coating method of a solution containing a polymer include various coating methods such as a dip method, a spray method, a pad printing method and a die coating method. From the viewpoint of the water wettability, the lubricity and shortening of the production process, a dip method and a spray method are preferable. From the viewpoint of the water wettability, the lubricity and shortening of the production process, a method in which a substrate is immersed in a solution containing a polymer and then heated is particularly preferable. Examples of the heating method include, but are not particularly limited to, an autoclave method, a hot air method, a dry heat method, a flame method and the like, and an autoclave method is preferable when heating is performed at a temperature exceeding 100°C.

If the heating temperature is too low, a surface of the medical device exhibiting satisfactory water wettability and lubricity cannot be obtained, and if the heating temperature is too high, the strength of the medical device itself is affected. Therefore, the heating temperature is preferably 50°C to 180°C, more preferably 60°C to 140°C, and more preferably 80°C to 130°C. If the heating time is too short, a surface of the medical device exhibiting satisfactory water wettability and lubricity cannot be obtained, and if it is too long, the strength of the medical device itself is affected. Therefore, the heating time is preferably 5 to 300 minutes, more preferably 10 minutes to 200 minutes, and still more preferably 15 minutes to 100 minutes. Any upper limit and any lower limit of the heating temperature and time may be used in combination.

When the method of immersing and heating is used, the initial pH of the solution is preferably in a range of 2.0 to **6.8,** more preferably 2.0 to 4.0, and most preferably 2.5 to **3.5,** since the solution does not become turbid and the surface of the obtained medical device has satisfactory transparency. Any upper limit and any lower limit may be used in combination.

One type of a carboxylic acid group-containing polymer and one type of a copolymer having an amide structure may form a layer on the surface of the substrate in one step. However, it is preferable to form a layer on the surface of the substrate in separate steps in that more durable surface wettability can be imparted.

The method for producing a medical device of the present invention preferably includes the following steps. Namely, the method preferably includes:

a step of disposing the substrate in a solution containing one type of the carboxylic acid group-containing polymer and heating at 50°C or higher, and

a step of disposing the substrate subjected to the step of heating at 50°C or higher in a solution containing a copolymer having an amide structure whose initial pH is adjusted in a range of 2 to 6, and heating at 60°C or higher.

In the second step, it is more preferable to heat at 80°C or higher in a solution containing a copolymer having an amide structure whose initial pH is adjusted in a range of 2 to 4, in that a copolymer complex having an amide structure can be formed on the carboxylic acid group-containing polymer layer, and a long-term durable layer can be formed.

The pH of the solution containing a polymer can be measured using a pH meter (for example, pH meter: KR5E (AS ONE Corporation)). The initial pH of the solution containing a polymer is the value of pH measured after the polymer and all other additives are added to the solution and the solution is stirred with a rotor for 2 hours at room temperature (23 to 25°C) to make it uniform. The value of pH is rounded off to the first decimal place.

After the heat treatment, the substrate having a polymer layer formed on the surface thereof may be immersed in a solution containing no polymer, and then the same heat treatment as mentioned above, radiation irradiation or the like may be further performed. In this case, it is possible to use, as the radiation, preferably various ion rays, electron beams, positron rays, X-rays, γ-rays and neutron rays, more preferably electron beams and γ-rays, and most preferably γ-rays.

<Wet Value Half-Life>

In the medical device of the present invention, the wet value half-life derived from the following equation (I) and equation (II) is preferably 160 seconds or more, more preferably 180 seconds or more, and particularly preferably 200 seconds or more, in that the surface wettability can be maintained for a long time even when used for a long time in the actual use environment. The upper limit of the surface wettability maintenance time is not particularly limited, and actual maintenance time of the surface wettability as the medical device is preferably 5,000 seconds or less:

$$Ln(F) = -At + B \qquad (I)$$

$$\text{Wet value half-life} = Ln(2/A) \qquad (II)$$

Where F represents a wet value (Fluidity factor) obtained by the MS-DWS method and t represents the time (seconds). Equation (I) is a linear approximation of a relationship between the logarithm of the wet value F and the time t. A represents an inclination of an approximate straight line of equation (I) and B represents the intercept.

A method for determining the wet value half-life by multi speckle-diffusing wave spectroscopy (hereinafter referred to as MS-DWS method) regarding the retention time of the surface wettability will be mentioned.

<Measurement Method by Multi Speckle-Diffusing Wave Spectroscopy>

A medical device, in hydrated state, hollowed out in a 15 mm circle shape is disposed directly below the place where the laser of the drying process evaluation device (Rheolaser Coating: sold by Sanyo Trading Co., Ltd.) is transmitted. Then, the measurement was started 10 seconds after water droplets on the surface were removed. Fluidity factor (wet value) by the MS-DWS method was measured for 10 minutes. When the medical device in hydrated state had a

lens shape with a diameter of 12 mm to 15 mm, it was placed on an aluminum pedestal as shown in FIGS. 1 and 2, and then the Fluidity factor (wet value) was measured for 10 minutes in the same manner. The measurement conditions are shown below. A sample was installed so that the distance from the bottom of the laser head to the measurement sample was 13 cm, and the laser head and the measurement sample (including the aluminum pedestal) were enclosed by a cardboard box having a width of 25 cm, a height of 27 cm and a depth of 35 cm in four directions other than the laser head surface and bottom surface to thereby block light and wind, and then the measurement was performed at 25°C and humidity of 65%.

<How to Determine Wet Value Half-Life>

FIG. 3 shows the change in wet value (Fluidity factor) with time measured by the MS-DWS method. F represents a wet value (Fluidity factor) and t represents the time (seconds). From this curve, the following linear approximation equation (I) is determined using Excel (software manufactured by Microsoft Corporation):

$$\mathrm{Ln}(F) = -At + B \qquad (I)$$

$$\text{wet value half-life} = \mathrm{Ln}(2/A) \qquad (II)$$

where F represents a wet value (Fluidity factor) obtained by the MS-DWS method and t represents the time (seconds); equation (I) is a linear approximation of a relationship between the logarithm of the wet value F and the time **t; A** represents an inclination of an approximate straight line of equation (I) and B represents the intercept. The measurement is performed three times, and the average of an inclination A is calculated. The wet value half-life (seconds) is obtained from the average of the inclination A and the above equation (II).

When the medical device of the present invention is an ophthalmic lens, and particularly a soft contact lens, many people felt dry by wearing the contact lens for a long time, and it is particularly effective to maintain the surface wettability of the contact lens for a long time. When a medical electrode for skin also requires a long-time measurement, it is particularly effective to maintain the surface wettability for a long time in order to maintain contact resistance with the skin.

<Dry Elastic Modulus Ratio>

The medical device of the present invention preferably has a dry elastic modulus ratio represented by the following equation (III) in a range of 0.1 or more and 10 or less. The definitions of "hydrated state" and "dry state" are as mentioned above.

Dry elastic modulus ratio = tensile elastic modulus in dry state/tensile elastic modulus in hydrated state (III)

The medical device of the present invention preferably has a small change in tensile elastic modulus with drying. It is preferable that the tensile elastic modulus in hydrated state is in the above-mentioned preferable range, and the change in the tensile elastic modulus before and after drying is small. From the viewpoint of having flexibility to the extent that the medical device does not easily damage the human body even when it is completely dried, the dry elastic modulus ratio represented by the equation (III) is preferably 10 or less. The dry elastic modulus is preferably 0.1 or more from the viewpoint that the handleability in hydrated state can be maintained without significantly reducing the elastic modulus by drying.

The lower limit of the dry modulus ratio is preferably 0.1, more preferably 0.5, still more preferably 1, yet more preferably 1.2, and particularly preferably 1.5. The upper limit is preferably 10, more preferably 7, still more preferably 5, yet more preferably 3, and particularly preferably 2.5. Any upper limit value and any lower limit value may be used in combination.

The tensile elastic modulus in hydrated state and the tensile elastic modulus in dry state for obtaining the above-mentioned dry elastic modulus ratio will be described below.

<Tensile Elastic Modulus In Hydrated State >

The medical device of the present invention preferably has a tensile elastic modulus in hydrated state in a range of 0.1 MPa or more and 1.5 MPa or less. If the tensile elastic modulus in hydrated state is 0.1 MPa or more, the shape of the medical device can be maintained, and if the tensile elastic modulus in hydrated state is 1.5 MPa or less, eyeballs may be stimulated during wearing leading to deterioration of comfort. The lower limit is preferably 0.1 MPa or more, more preferably 0.25 MPa, still more preferably 0.35 MPa, yet more preferably 0.4 MPa, and particularly preferably 0.48 MPa. The upper limit is preferably 1.5 MPa, more preferably 1.4 MPa, still more preferably 1.2 MPa, and yet more preferably 1

MPa. Any upper limit value and any lower limit value may be used in combination. The tensile elastic modulus in hydrated state is determined by the following procedure: within 10 seconds after a test piece is taken out of a phosphate buffered saline solution, the phosphate buffered saline solution on the surface is lightly wiped off with a clean cloth under the conditions of room temperature of 20°C and humidity of 50%, and then, within 1 minute, the measurement is performed using a tensile tester under the conditions of room temperature of 20°C and humidity of 50%.

<Tensile Elastic Modulus In Dry State>

The medical device of the present invention preferably has a tensile elastic modulus in dry state in a range of 0.1 MPa or more and 3.5 MPa or less. "Dry state" means a state of being dried in a vacuum dryer at 40°C for 16 hours or more. If the tensile elastic modulus in dry state is 0.1 MPa or more, sufficient followability can be easily obtained, and if the tensile elastic modulus in dry state is 3.5 MPa or less, there is little risk that a part of the human body such as eyeballs is stimulated with drying leading to deterioration of comfort or damage. The lower limit is preferably 0.35 MPa, more preferably 0.4 MPa, still more preferably 0.55 MPa, yet more preferably 0.7 MPa, and further preferably 0.8 MPa. The upper limit is preferably 3.5 MPa, more preferably 2.8 MPa, still more preferably 2.4 MPa, still more preferably 2 MPa, yet more preferably 1.7 MPa, and further preferably 1.5 MPa. Any upper limit value and any lower limit value may be used in combination. The tensile elastic modulus in dry state can be measured using a tensile tester within 5 minutes after being taken out of the dryer under the conditions of room temperature of 20°C and humidity of 50%.

<Embodiment of Medical Device>

The medical device of the present invention is suitably used as an ophthalmic lens, a dermal covering material, a wound dressing material, a skin protection material, a skin medicine carrier, an infusion tube, a gas delivery tube, a drain tube, a blood circuit, a covering tube, a catheter, a stent, a sheath, a tube connector, an access port or an endoscopic dressing material.

In a preferred embodiment of the invention, the medical device of the invention may be tubular. As an example of a tubular device, the medical device can be preferably used as an infusion tube, a gas delivery tube, a drain tube, a blood circuit, a covering tube, a catheter, a stent, a sheath, a tube connector or an access port.

In another preferred embodiment of the present invention, the medical device may be in the form of a sheet or a film. Specifically, the medical device can also be preferably used as a dermal covering material, a wound dressing material, a skin protection material, a skin medicine carrier or an endoscopic dressing material.

In yet another preferred embodiment of the invention, the medical device may have a storage container shape. Specifically, the medical device can also be preferably used as a drug carrier, a cuff or a drainage bag.

In yet another preferred embodiment of the invention, the medical device may have a lens shape. Specifically, the medical device can also be preferably used as ophthalmic lenses such as an intraocular lens, an artificial cornea, a corneal inlay, a corneal onlay and a spectacle lens.

Hereinafter, the present invention will be specifically described with reference to Examples, but the present invention is not limited thereto.

[Examples]

Preferred embodiments of the present invention will be described by way of Examples.

Abbreviations of the monomers used in the examples are mentioned below.

MEA: 2-Methoxyethyl acrylate, manufactured by Tokyo Chemical Industry Co., Ltd.

EEMA: 2-Ethoxyethyl methacrylate, manufactured by Tokyo Chemical Industry Co., Ltd.

DMAA: N,N-dimethylacrylamide, manufactured by Tokyo Chemical Industry Co., Ltd.

164B: Difunctional silicone monomer, X-22-164B ($\alpha$,$\omega$-di-(3-methacryloxy-propyl)-polydimethylsiloxane), functional group equivalent: 1,600, manufactured by Shin-Etsu Chemical Co., Ltd.

FM0711: Linear polymerizable silicone compound "Silaplane" (registered trademark) (polydimethylsiloxane monomethacrylate), average molecular weight: 1000, manufactured by JNC Corporation, FM0711 is a compound corresponding to the general formula (1) in which $R^3$, $R^5$, $R^6$, $R^7$ and $R^8$ are a methyl group, $R^4$ is an n-propylene group, $R^9$ is an n-butyl group, and k is 10.

NB: Ultraviolet absorber 2-(2'-hydroxy-5'-methacryloyloxyethylphenyl)-2H-benzotriazole, manufactured by Tokyo Chemical Industry Co., Ltd.

RB: Colorant Reactive Blue 246 (1,4-bis[4-(2-methacryloxyethyl)phenylamino]anthraquinone), manufactured by Arran Chemical Co., LTD.

IC-819: Photoinitiator "IRGACURE" (registered trademark) 819 (phenylbis(2,4,6-trimethylbenzoyl)phosphine oxide)

(acylphosphine oxide-based polymerization initiator), manufactured by Ciba Specialty Chemicals Inc.
MBF: Methyl benzoylformate (intramolecular hydrogen abstraction type polymerization initiator) manufactured by Tokyo Chemical Industry Co., Ltd.
TAA: Non-polymerizable solvent, tert-amyl alcohol, manufactured by Tokyo Chemical Industry Co., Ltd.

<Fabrication of Substrate A>

2.2 Gram (g) of MEA, 4.3 g of FM0711, 2.5 g of DMAA, 1.0 g of 164B, 0.05 g of NB, 0.002 g of RB, 0.02 g of IC-819 (the content of an acylphosphine oxide-based polymerization initiator in a composition for forming a substrate is 0.14% by mass) and 4.0 g of TAA were well mixed. This mixture was filtered through a membrane filter (0.45 μm) to remove an insoluble substance to obtain a monomer composition. This monomer composition was injected into a contact lens mold made of transparent resin (polypropylene on the base curve side, cyclic polyolefin on the front curve side) having a diameter of 14 mm with a base curve of 8.6 mm and a thickness of 100 nm, and then polymerized by irradiation with light (1.01 $mW/cm^2$, 20 minutes) in a nitrogen atmosphere using a fluorescent lamp (Toshiba, FL-6D, daylight color, 6 W, 4 tubes). After polymerization, the entire mold was immersed in a mixed solution of isopropyl alcohol and water in a ratio of 55:45 (volume ratio before mixing) and heated at 60°C for 1 hour to remove a contact lens-shaped molded body from the mold. Thereafter, the molded body thus obtained was immersed in fresh isopropyl alcohol and water in a ratio of 55:45 (volume ratio before mixing) and heated at 60°C for 3 hours, followed by extraction.

The molded product thus obtained after extraction was immersed in a mixed solution of isopropyl alcohol and water in a ratio of 3:7 (volume ratio before mixing) at room temperature for 30 minutes, immersed in clean RO water and then left to stand overnight and stored to obtain a substrate A. The content of a 2-methoxyethyl group in the substrate is 10% by mass.

<Fabrication of Substrate B>

In the same manner as in fabrication of the substrate A, except that the amount of MEA was changed to 0.8 g and the amount of FM0711 was changed to 5.7 g, a substrate B was obtained. The content of a 2-methoxyethyl group in the substrate is 4% by mass.

<Fabrication of Substrate C>

In the same manner as in fabrication of the substrate A, except that the amount of MEA was changed to 2.8 g and the amount of FM0711 was changed to 3.7 g, a substrate C was obtained. The content of a 2-methoxyethyl group in the substrate is 13% by mass.

<Fabrication of Substrate D>

In the same manner as in fabrication of the substrate A, except that the amount of MEA was changed to 6.5 g and FM0711 was not used, a substrate D was obtained. The content of a 2-methoxyethyl group in the substrate is 30% by mass.

<Fabrication of Substrate E>

In the same manner as in fabrication of the substrate A, except that MEA was not used and the amount of FM0711 was changed to 6.5 g, a substrate E was obtained. The content of a 2-methoxyethyl group in the substrate is 0% by mass.

<Fabrication of Substrate F>

In the same manner as in the substrate A, except that the amount of DMAA was changed to 1.5 g and the amount of FM0711 was changed to 5.3 g, a substrate F was obtained. The content of a 2-methoxyethyl group in the substrate is 10% by mass.

<Fabrication of Substrate G>

In the same manner as in the substrate A, except that the amount of DMAA was changed to 5.0 g and the amount of FM0711 was changed to 1.8 g, a substrate G was obtained. The content of a 2-methoxyethyl group in the substrate is 10% by mass.

<Fabrication of Substrate H>

In the same manner as in the substrate A, except that the amount of MEA was changed to 3.0 g, the amount of DMAA was changed to 2.5 g, the amount of 164B was changed to 1.0 g, and 3.5 g of 2-hydroxymethyl methacrylate was used, a substrate H was obtained. The content of a 2-methoxyethyl group in the substrate is 10% by mass.

<Fabrication of Substrate I>

In the same manner as in the substrate A, except that MEA was not used and 2.2 g of EEMA was used, a substrate H was obtained. The content of a 2-ethoxyethyl group in the substrate is 12% by mass.

<Fabrication of Substrate J>

In the same manner as in the substrate A, except that the amount of TAA was changed to 2 g, a substrate J was fabricated. The content of IC-819 as an acylphosphine oxide-based polymerization initiator in a composition for forming a substrate is 0.17% by mass. The content of an intramolecular hydrogen abstraction type polymerization initiator in a composition for forming a substrate is 0% by mass.

<Fabrication of Substrate K>

In the same manner as in the substrate A, except that the amount of TAA was changed to 2 g and, in addition to 0.02 g of IC-819 (acylphosphine oxide-based polymerization initiator), 0.05 g of MBF (intramolecular hydrogen abstraction type polymerization initiator) was used, a substrate K was obtained. The content of an acylphosphine oxide-based polymerization initiator in a composition for forming a substrate is 0.16% by mass. The content of an intramolecular hydrogen abstraction type polymerization initiator in a composition for forming a substrate is 0.41% by mass.

<Fabrication of Substrate L>

In the same manner as in the substrate A, except that the amount of TAA was changed to 2 g and, in addition to 0.02 g of IC-819 (acylphosphine oxide-based polymerization initiator), 0.4 g of MBF (intramolecular hydrogen abstraction type polymerization initiator) was used, a substrate L was obtained. The content of an acylphosphine oxide-based polymerization initiator in a composition for forming a substrate is 0.16% by mass. The content of an intramolecular hydrogen abstraction type polymerization initiator in a composition for forming a substrate is 3.21% by mass.

<Fabrication of Substrate M>

In the same manner as in the substrate A, except that the amount of TAA was changed to 2 g and, in addition to 0.02 g of IC-819 (acylphosphine oxide-based polymerization initiator), 2.5 g of MBF (intramolecular hydrogen abstraction type polymerization initiator) was used and 2 g of TAA was used, a substrate M was obtained. The content of an acylphosphine oxide-based polymerization initiator in a composition for forming a substrate is 0.14% by mass. The content of an intramolecular hydrogen abstraction type polymerization initiator in a composition for forming a substrate is 17.2% by mass.

<Formation of Substrate N>

In the same manner as in the substrate A, except that the amount of TAA was changed to 2 g, the amount of IC-819 (acylphosphine oxide-based polymerization initiator) was changed to 0.05 g, and 0.05 g of MBF (intramolecular hydrogen abstraction type polymerization initiator) was used, a substrate N was obtained. The content of an acylphosphine oxide-based polymerization initiator in a composition for forming a substrate is 0.41% by mass. The content of an intramolecular hydrogen abstraction type polymerization initiator in a composition for forming a substrate is 0.41% by mass.

<Fabrication of Substrate O>

In the same manner as in the substrate A, except that the amount of TAA was changed to 2 g, the amount of IC-819 (acylphosphine oxide-based polymerization initiator) was changed to 0.08 g, and 0.05 g of MBF (intramolecular hydrogen abstraction type polymerization initiator) was used, a substrate O was obtained. The content of an acylphosphine oxide-based polymerization initiator in a composition for forming a substrate is 0.66% by mass. The content of an intramolecular hydrogen abstraction type polymerization initiator in a composition for forming a substrate is 0.41% by mass.

<Fabrication of Substrate P>

In the same manner as in the substrate A, except that the amount of TAA was changed to 2 g and, in addition to 0.02 g of IC-819 (acylphosphine oxide-based polymerization initiator), 0.02 g of MBF (intramolecular hydrogen abstraction type polymerization initiator) was used, a substrate P was obtained. The content of an acylphosphine oxide-based polymerization initiator in a composition for forming a substrate is 0.17% by mass. The content of an intramolecular hydrogen abstraction type polymerization initiator in a composition for forming a substrate is 0.17% by mass.

<Fabrication of Substrate Q>

In the same manner as in the substrate A, except that the amount of TAA was changed to 2 g, the amount of IC-819 (acylphosphine oxide-based polymerization initiator) was changed to 0.1 g, and 4 g of MBF (intramolecular hydrogen abstraction type polymerization initiator) was used, a substrate Q was obtained. The content of an acylphosphine oxide-based polymerization initiator in a composition for forming a substrate is 0.62% by mass. The content of an intramolecular hydrogen abstraction type polymerization initiator in a composition for forming a substrate is 24.8% by mass.

[Example 1]

As the first treatment, the substrate A was placed in an aqueous 0.5% by mass polyacrylic acid ("Sokalan" (registered trademark) PA110S, weight-average molecular weight of 250,000, manufactured by BASF Corporation) solution and heating was performed in a hot air oven at 60°C for 30 minutes. Thereafter, the substrate was taken out of the aqueous solution and immersed in 100 mL of pure water, followed by washing with shaking. As the second treatment, sulfuric acid was added little by little to a 1.2% by mass acrylic acid/N,N-dimethylacrylamide copolymer (copolymerization ratio of 1/9 [molar ratio], weight-average molecular weight of 1,040,000) phosphate buffered saline solution to adjust the pH to 3.0, and the substrate was placed in this polymer solution, and then heated in an autoclave at 121°C for 30 minutes. Thereafter, the substrate was taken out of the polymer solution and immersed in 100 mL of pure water, followed by washing with shaking to obtain a contact lens having a polymer layer. The pH of the aqueous polymer solution was adjusted while measuring with a pH meter: KR5E (manufactured by AS ONE Corporation).

The contact lenses thus obtained were subjected to the following evaluations (1) to (8). The results are summarized in Table 1.

<Measurement of Molecular Weight>

The molecular weight of the polymer used was measured under the following conditions. Herein, the weight average molecular weight is sometimes referred to Mw. The molecular weight of 1,000 is sometimes referred to as 1 kD.

(GPC Measurement Conditions)

Apparatus: Prominence GPC system manufactured by Shimadzu Corporation
Pump: LC-20AD
Autosampler: SIL-20AHT
Column oven: CTO-20A
Detector: RID-10A
Column: GMPXW, manufactured by Tosoh Corporation (7.8 mm in inner diameter × 30 cm, particle diameter of 13 μm)
Solvent: water/methanol = 1/1 (0.1 N lithium nitrate is added)
Flow rate: 0.5 mL/minute
Measurement time: 30 minutes
Sample concentration: 0.1 by mass
Sample injection amount: 20 μL
Standard sample: Polyethylene oxide standard sample, manufactured by Agilent Technologies, Inc. (0.1 kD to 1,258 kD)

<Method for Fabrication of Phosphate Buffered Saline Solution>

In a 2 L Erlenmeyer flask, 16 g of sodium chloride, 0.4 g of potassium chloride, 2.88 g of disodium hydrogen

phosphate, 0.48 g of potassium dihydrogen phosphate and 2 L of RO water were weighed. A stirrer was put in the flask and the mixture was well stirred, and after confirming that the reagent is dissolved, filtration was performed using a polyether sulfone filter (250ML EXPRESS PLUS PES 22UM 45MM, manufactured by Merck KGaA) and a vacuum pump to obtain a phosphate buffered saline solution.

(1) Measurement of Water Content

The mass in hydrated state (W1) and the mass in dry state (W2) of the contact lens were measured. Based on the respective measurement results, the water content was calculated by the following equation.

$$\text{Water content } (\%) = (W1 - W2) \times 100/W1$$

"hydrated state" in the present invention means states obtained by the following operations. A contact lens is immersed in a phosphate buffered saline solution at 25°C for 1 day or more. After taking the contact lens out of the phosphate buffered saline solution, the contact lens is immediately sandwiched between two pieces of "Haize" Gauze VP-150 (manufactured by OZU CORPORATION) from above and below, and then water on the surface was removed by lightly pressing. Once again, the contact lens was sandwiched between two pieces of "Haize" Gauze VP-150 (manufactured by OZU CORPORATION) from above and below and then water on the surface was removed by lightly pressing. The state of the contact lens immediately after these operations was defined as a state in hydrated state. Regarding "dry state" of the contact lens, a state immediately after the contact lens is dried in a vacuum dryer at 40°C for 16 hours or more and taken out of the vacuum dryer was defined as a state in dry state.

(2) Evaluation of Water Wettability (Initial Water Wettability)

A contact lens was immersed in a phosphate buffered saline solution in a beaker at room temperature (25°C) for 24 hours or more. Thereafter, when the contact lens was pulled up from the phosphate buffered saline solution and held in the air so that the radial direction was vertical, the state of the surface of the contact lens was visually observed, and the retention time of the liquid film on the surface was measured. Here, the retention time of the liquid film is the time from the point when the contact lens begins to be held vertically to the point when a portion of the phosphate buffered saline solution film covering the lens convex surface breaks off and the lens surface is no longer completely covered by the liquid film.

(3) Durability Test of Surface Wettability

A contact lens was immersed in a phosphate buffered saline solution in a beaker at room temperature (25°C) for 24 hours or more. Thereafter, the contact lenses were pulled up from the phosphate buffered saline solution, and after scrubbing the surface 30 times back and forth with the thumb and the index finger, the contact lens was immersed again in the phosphate buffered saline solution and then stored. The next day and thereafter, the same scrubbing was performed for a total of 7 days (scrubbing 30 times back and forth/day). Thereafter, when the contact lens was pulled up from the phosphate buffered saline solution and held in the air so that the radial direction was vertical, the state of the surface of the contact lens was visually observed, and the retention time of the liquid film on the surface was measured.

(4) Durability Test of Lubricity

The contact lens after subjecting to the test (3) was immersed in a phosphate buffered saline solution in a beaker at room temperature for 1 minute or more. The contact lens was pulled up from the phosphate buffered saline solution and subjected to sensory evaluation when rubbing with a human finger five times.

A: There is extremely excellent lubricity.
B: There is lubricity intermediate between A and C.
C: There is moderate lubricity.
D: Almost no lubricity (intermediate between C and E).
E: No lubricity.

(5) Wet Value Half-Life (measurement of wet value and how to determine wet value half-life regarding retention time of surface wettability)

A contact lens, in hydrated state, hollowed out in a 13 mm circle shape is disposed directly below the place where the laser of the drying process evaluation device (Rheolaser Coating: sold by Sanyo Trading Co., Ltd.) is transmitted.

Then, the measurement was started 10 seconds after water droplets on the surface were removed using "Haize" Gauze VP-150: manufactured by OZU CORPORATION). Fluidity factor (wet value) by the MS-DWS method was measured for 10 minutes. When the contact lens had a lens shape with a diameter of 12 mm to 15 mm, it was placed on an aluminum pedestal as shown in FIGS. 1 and 2, and then the Fluidity factor (wet value) was measured for 10 minutes in the same manner. The measurement conditions are shown below. A sample was installed so that the distance from the bottom of the laser head to the measurement sample was 13 cm, and the laser head and the measurement sample (including the aluminum pedestal) were enclosed by a cardboard box having a width of 25 cm, a height of 27 cm and a depth of 35 cm in four directions other than the laser head surface and bottom surface to thereby block light and wind, and then the measurement was performed at 25°C and humidity of 65%.

Next, how to obtain the wet value half-life is shown. FIG. 3 shows the change in wet value (Fluidity factor) with time measured by the MS-DWS method. F represents a wet value (Fluidity factor) and t represents the time (seconds). From this curve, the following linear approximation equation (I) was determined using Excel (software manufactured by Microsoft Corporation):

$$\mathrm{Ln}(F) = -At + B \qquad (I)$$

$$\text{wet value half-life} = \mathrm{Ln}(2/A) \qquad (II)$$

Where F represents a wet value (Fluidity factor) obtained by the MS-DWS method and t represents the time (seconds). Equation (I) is a linear approximation of a relationship between the logarithm of the wet value F and the time t. A represents an inclination of an approximate straight line of equation (I) and B represents the intercept. The measurement was performed three times, and the average of an inclination A was calculated. The wet value half-life (seconds) was obtained from the average of the inclination A and the above equation (II).

(6) Tensile Elastic Modulus In Hydrated State

An array type sample having a width of 5 mm at the narrowest part was cut out from a contact lens in a water-containing state, and then the thickness was measured using an ABC Digimatic Indicator (ID-C112, manufactured by Mitutoyo Corporation). Next, the tensile elastic modulus was measured by pulling with TENSILON (RTM-100, manufactured by Toyo Baldwin Co., Ltd., crosshead speed of 100 mm/min) at 25°C and humidity of 50% with a test length of 5 mm centered on the narrowest part of the array type sample.

(7) Tensile Elastic Modulus In Dry State

In the same manner as in "(6) Tensile Elastic Modulus In Hydrated State", except that a contact lens in a dry state is used in place of a contact lens in a water-containing state, the tensile elastic modulus in dry state was measured.

(8) Dry Elastic Modulus Ratio

The dry elastic modulus ratio was determined from the tensile elastic modulus in hydrated state measured in (6) and the tensile elastic modulus in dry state measured in (7) using the following equation (III).

Dry elastic modulus ratio = tensile elastic modulus in dry state/tensile elastic modulus in hydrated state     Equation (III).

[Examples 2 to 7, Comparative Examples 1 to 10]

In the same manner as in Example 1, except that the substrate used and the polymer solution used for forming a surface polymer layer were those shown in Tables 1 and 2, contact lenses were obtained. The results are shown in Table 1 and Table 2.

[Table 1]

| | Substrated used | Formation of surface polymer layer | | (1) Measurement of water content [%] | (2) Evaluation of water wettability [sec] | (3) Durability test of surface wettability [sec] | (4) Durability test of lubricity | (5) Wet value half-life [sec] | (6) Tensile elastic modulus in hydrated state [MPa] | (7) Tensile elastic modulus in dry state [MPa] | (8) Dry elastic modulus ratio |
|---|---|---|---|---|---|---|---|---|---|---|---|
| | | Solution used in first treatment | Solution used in second treatment | | | | | | | | |
| Example 1 | Substrate A | Solution A1 | Solution B1 | 34.2 | 60 | 60 | A | 241 | 1.8 | 2.7 | 1.5 |
| Example 2 | Substrate B | Solution A1 | Solution B1 | 28.8 | 60 | 60 | B | 181 | 1.6 | 3.5 | 2.2 |
| Example 3 | Substrate C | Solution A1 | Solution B1 | 35.3 | 60 | 60 | A | 205 | 1.6 | 3.2 | 2.0 |
| Example 4 | Substrate D | Solution A1 | Solution B1 | 37.2 | 60 | 60(*1) | A | 178 | 1.2 | 3.0 | 2.5 |
| Example 5 | Substrate A | Solution A2 | Solution B1 | 34.2 | 60 | 32 | B | 204 | *2 | *2 | *2 |
| Example 6 | Substrate A | Solution A3 | Solution B1 | 34.2 | 60 | 5 | B | 180 | *2 | *2 | *2 |
| Example 7 | Substrate I | Solution A1 | Solution B1 | 31 | 60 | 60 | A | 261 | 1.7 | 3.2 | 1.9 |

*1: The substrate was torn during the test.
*2: Not measured

[Table 2]

| | Substrate used | Formation of surface polymer layer | | (1) Measurement of water content [%] | (2) Evaluation of water wettability [sec] | (3) Durability test of surface wettability [sec] | (4) Durability test of lubricity | (5) Wet value half-life [sec] | (6) Tensile elastic modulus in hydrated state [MPa] | (7) Tensile elastic modulus in dry state [MPa] | (8) Dry elastic modulus ratio |
|---|---|---|---|---|---|---|---|---|---|---|---|
| | | Solution used in first treatment | Solution used in second treatment | | | | | | | | |
| Comparative Example 1 | Substrate E | Solution A1 | Solution B1 | 27.9 | 60 | 60 | B | 120 | *2 | *2 | *2 |
| Comparative Example 2 | Substrate F | Solution A1 | Solution B1 | 20.2 | 60 | 60 | B | 108 | 2.2 | 22.4 | 10.2 |
| Comparative Example 3 | Substrate A | None | Solution B1 | 33.4 | 5 | 3 | E | 121 | *2 | *2 | *2 |
| Comparative Example 4 | Substrate G | Solution A1 | Solution B1 | 52.1 | 60 | 52 | C | 132 | *2 | *2 | *2 |
| Comparative Example 5 | Substrate A | Solution A4 | Solution B1 | 32.8 | 10 | 8 | D | 92 | *2 | *2 | *2 |
| Comparative Example 6 | Substrate A | Solution A5 | Solution B1 | 32.1 | 12 | 3 | C | 111 | *2 | *2 | *2 |
| Comparative Example 7 | Substrate A | Solution A6 | Solution B1 | 33.5 | 8 | 2 | E | 80 | *2 | *2 | *2 |
| Comparative Example 8 | Substrate A | Solution A7 | Solution B1 | 42.2 | *1 | *1 | *1 | *1 | *2 | *2 | *2 |
| Comparative Example 9 | Substrate A | Solution A1 | Solution B2 | 33.2 | 60 | 32 | E | 152 | *2 | *2 | *2 |
| Comparative Example 10 | Substrate H | Solution A1 | Solution B1 | 54.2 | 60 | 60 | B | 200 | 1.7 | 9.9 | 5.8 |

*1: Evaluation was not performed since the substrate was swollen and deformed.
*2: Not measured

Solution A1: Aqueous 0.5% by mass polyacrylic acid solution (Mw = 250,000)
Solution A2: Aqueous 0.5% by mass polyacrylic acid solution (Mw = 25,000)
Solution A3: Aqueous 0.5% by mass polyacrylic acid solution (Mw = 8,000)
Solution A4: 1.0% by mass polyvinyl alcohol (Mw = 30,000)
Solution A5: 1.2% by mass acrylic acid/N,N-dimethylacrylamide copolymer (copolymerization ratio 2/1, Mw = 1,040,000)
Solution A6: Aqueous 1.0% by mass tamarind gum solution (Mw = 470, 000)
Solution A7: 1.0% by mass O-[2-hydroxy-3-(trimethylammonio)propyl]guar gum chloride (= Rhaball gum CG-M8M)
Solution B1: 1.2% by mass acrylic acid/N,N-dimethylacrylamide copolymer (copolymer ratio of 1/9, Mw = 1,040,000)
Solution B2: 1.2% by mass N,N-dimethylacrylamide polymer Mw = 53,000).

[Examples 8 to 15]

The substrates used were those shown in Table 3, and the surface polymer layer was formed in the same manner as in Example 1 to fabricate a contact lens-shaped medical device, and the following tests (9) to (11) were performed. The results are shown in Table 3.

(9) Measurement of Yellowness

Yellowness (YI) of contact lenses was measured using a color computer (SM-7-CH, Suga Test Instruments Co., Ltd.). The contact lens was placed in the measurement hole so that it is convex downward and is not tilted, and then the yellow value (YI) of transmitted light was measured by the 45/0 method. The smaller the value of YI, the lower the yellowness and the better the results, and the negative value is more preferable because the color is bluish.

(10) Number of Lenses Damaged or Deformed During Peeling to Extraction

When 16 lenses were fabricated in each of the steps of fabricating substrates J to Q, the number of lenses damaged or deformed in the process from peeling to extraction is shown in Table 3. The smaller the number, the less deformation and damage and the better the results.

(11) Strength Test

After scrubbing 10 contact lenses 50 times each with fingers on the palm, the contact lenses were folded in half and scrubbed 30 times with the thumb and the ring finger, followed by spreading and pulling with a light force. Table 3 shows the number of torn lenses out of 10 lenses. The smaller the number, the more the lens is resistant to tearing, which indicates satisfactory results.

[Table 3]

| | Substrate used | Acylphosphine oxide-based polymerization initiator [% by mass] | Intramolecular hydrogen abstraction type polymerization initiator [% by mass] | (9) Yellowness (YI) | (10) Number of lenses damaged or deformed during peeling to extraction/16 lenses | (11) Strength test/10 lenses |
|---|---|---|---|---|---|---|
| Example 8 | Substrate J | 0.17 | 0.00 | -4.1 | 9 lenses | 4 lenses |
| Example 9 | Substrate K | 0.16 | 0.41 | -3.8 | 0 lens | 1 lens |
| Example 10 | Substrate L | 0.16 | 3.21 | -3.4 | 0 lens | 1 lens |
| Example 11 | Substrate M | 0.14 | 17.2 | -1.2 | 2 lenses | 2 lenses |

(continued)

| | Substrate used | Acylphosphine oxide-based polymerization initiator [% by mass] | Intramolecular hydrogen abstraction type polymerization initiator [% by mass] | (9) Yellowness (YI) | (10) Number of lenses damaged or deformed during peeling to extraction/16 lenses | (11) Strength test/10 lenses |
|---|---|---|---|---|---|---|
| Example 12 | Substrate N | 0.41 | 0.41 | 0.2 | 0 lens | 1 lens |
| Example 13 | Substrate O | 0.66 | 0.41 | 7.2 | 2 lenses | 3 lenses |
| Example 14 | Substrate P | 0.17 | 0.17 | -4.2 | 10 lenses | 0 lens |
| Example 15 | Substrate Q | 0.62 | 24.8 | 9.0 | 5 lenses | 0 lens |

[Reference Signs List]

1: Aluminum pedestal

2: Lens-shaped medical device

## Claims

**1.** A medical device comprising a substrate and a polymer layer, wherein the polymer layer is provided on at least a part of a surface of the substrate and satisfies the following requirements:

(a) a water content of the medical device in hydrated state is in a range of 28% by mass or more and 50% by mass or less;
(b) the substrate has a 2-alkoxyethyl group; and
(c) the polymer layer contains one type of a carboxylic acid group-containing polymer and one type of a copolymer having an amide structure.

**2.** The medical device according to claim 1, wherein the 2-alkoxyethyl group is a 2-methoxyethyl group.

**3.** The medical device according to claim 1, wherein the 2-alkoxyethyl group is a 2-ethoxyethyl group.

**4.** The medical device according to any one of claims 1 to 3, wherein the carboxylic acid group-containing polymer has a weight-average molecular weight in a range of 10,000 or more and 500,000 or less.

**5.** The medical device according to any one of claims 1 to 4, wherein the substrate has the 2-alkoxyethyl group in a range of 5% by mass or more and 25% by mass or less.

**6.** The medical device according to any one of claims 1 to 5, wherein a wet value half-life derived from the following equation (I) and equation (II) is in a range of 160 seconds or more and 5,000 seconds or less:

$$\mathrm{Ln}(F) = -At + B \qquad (I)$$

$$\text{wet value half-life} = \mathrm{Ln}(2/A) \qquad (II)$$

where F represents a wet value (Fluidity factor) obtained by the MS-DWS method and t represents the time (seconds);

equation (I) is a linear approximation of a relationship between the logarithm of the wet value F and the time t; A represents an inclination of an approximate straight line of equation (I) and B represents the intercept.

7. The medical device according to any one of claims 1 to 6, wherein a dry elastic modulus ratio represented by the following equation (III) is in a range of 0.1 or more and 10 or less.

$$\text{Dry elastic modulus ratio} = \text{tensile elastic modulus in dry state}/\text{tensile elastic modulus in hydrated state} \quad \text{(III)}$$

8. The medical device according to claim 7, wherein the tensile elastic modulus in dry state is in a range of 0.1 MPa or more and 3.5 MPa or less.

9. The medical device according to any one of claims 1 to 8, wherein the substrate includes a unit derived from the following general formula (1):

[Chemical Formula 1]

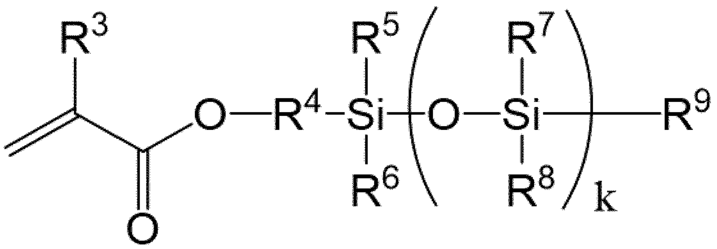

··(1)

wherein, in the general formula (1), $R^3$ represents a hydrogen atom or a methyl group; $R^4$ represents a divalent organic group having 1 to 20 carbon atoms; $R^5$ to $R^8$ each independently represents an alkyl group having 1 to 20 carbon atoms or an aryl group having 6 to 20 carbon atoms; $R^9$ represents an optionally substituted alkyl group having 1 to 20 carbon atoms or an optionally substituted aryl group having 6 to 20 carbon atoms; and k represent an integer in a range of 1 to 200.

10. The medical device according to any one of claims 1 to 9, wherein the carboxylic acid group-containing polymer is one or more types selected from the group consisting of polymethacrylic acid, polyacrylic acid, polyvinylbenzoic acid, poly(thiophene-3-acetic acid), polymaleic acid, poly(meth)acryloyloxyethyl-succinic acid, poly(meth)acryloyloxyethyl hexahydrophthalic acid, poly(meth)acryloyloxyethyl-phthalic acid, poly(meth)acryloyloxyethyl-2-hydroxyethyl-phthalic acid, polynorbornenecarboxylic acid, poly(meth)acryloyloxyethyl succinate, and salts thereof.

11. The medical device according to any one of claims 1 to 10, wherein the copolymer having an amide structure is one or more types selected from the group consisting of a (meth)acrylic acid/N-vinylpyrrolidone copolymer, a (meth)acrylic acid/N,N-dimethylacrylamide copolymer, a 2-acrylamide-2-methylpropanesulfonic acid/N-vinylpyrrolidone copolymer and a 2-acrylamide-2-methylpropanesulfonic acid/N,N-dimethylacrylamide copolymer.

12. The medical device according to any one of claims 1 to 11, wherein the substrate is a cured product of a composition for forming a substrate, wherein
the composition for forming a substrate includes a polymerizable compound having a 2-alkoxyethyl group, an acylphosphine oxide-based polymerization initiator and an intramolecular hydrogen abstraction type polymerization initiator, and includes the acylphosphine oxide-based polymerization initiator in a range of 0.05% by mass to 0.4% by mass and the intramolecular hydrogen abstraction type polymerization initiator in a range of 0.2% by mass to 20% by mass.

13. The medical device according to any one of claim 12, wherein the intramolecular hydrogen abstraction type polymerization initiator is methyl benzoylformate.

14. The medical device according to any one of claims 1 to 13, which is an ophthalmic lens, a dermal covering material, a wound dressing material, a skin protection material, a skin medicine carrier, an infusion tube, a gas delivery tube, a drain tube, a blood circuit, a covering tube, a catheter, a stent, a sheath, a tube connector, an access port or an

endoscopic dressing material.

**15.** A method for producing the medical device according to any one of claims 1 to 14, which comprises:

a step of disposing the substrate in a solution containing one type of the carboxylic acid group-containing polymer and heating at 50°C or higher, and
a step of disposing the substrate subjected to the step of heating at 50°C or higher in a solution containing a copolymer having an amide structure whose initial pH is adjusted in a range of 2 to 6, and heating at 60°C or higher.

## Patentansprüche

**1.** Medizinische Vorrichtung, umfassend ein Substrat und eine Polymerschicht, wobei die Polymerschicht auf mindestens einem Teil einer Oberfläche des Substrats bereitgestellt ist und die folgenden Anforderungen erfüllt:

(a) ein Wassergehalt der medizinischen Vorrichtung in hydratisiertem Zustand liegt in einem Bereich von 28 Massen-% oder mehr und 50 Massen-% oder weniger;
(b) das Substrat weist eine 2-Alkoxyethylgruppe auf; und
(c) die Polymerschicht enthält eine Art eines Carbonsäuregruppe-enthaltenden Polymers und eine Art eines Copolymers mit einer Amidstruktur.

**2.** Medizinische Vorrichtung gemäß Anspruch 1, wobei die 2-Alkoxyethylgruppe eine 2-Methoxyethylgruppe ist.

**3.** Medizinische Vorrichtung gemäß Anspruch 1, wobei die 2-Alkoxyethylgruppe eine 2-Ethoxyethylgruppe ist.

**4.** Medizinische Vorrichtung gemäß einem der Ansprüche 1 bis 3, wobei das Carbonsäuregruppe-enthaltende Polymer ein gewichtsgemitteltes Molekulargewicht in einem Bereich von 10.000 oder mehr und 500.000 oder weniger aufweist.

**5.** Medizinische Vorrichtung gemäß einem der Ansprüche 1 bis 4, wobei das Substrat die 2-Alkoxyethylgruppe in einem Bereich von 5 Massen-% oder mehr und 25 Massen-% oder weniger aufweist.

**6.** Medizinische Vorrichtung gemäß einem der Ansprüche 1 bis 5, wobei eine aus der folgenden Gleichung (I) und Gleichung (II) abgeleitete Nasswert-Halbwertszeit in einem Bereich von 160 Sekunden oder mehr und 5.000 Sekunden oder weniger liegt:

$$\mathrm{Ln}(F) = -At + B \quad (I)$$

$$\text{Nasswert-Halbwertszeit} = \mathrm{Ln}(2/A) \quad (II),$$

wobei F einen durch die MS-DWS-Methode ermittelten Nasswert (Fluiditätsfaktor) darstellt und t die Zeit (Sekunden) darstellt;
wobei Gleichung (I) eine lineare Näherung einer Beziehung zwischen dem Logarithmus des Nasswerts F und der Zeit t ist; wobei A eine Neigung einer näherungsweise geraden Linie von Gleichung (I) darstellt und B den Achsenabschnitt darstellt.

**7.** Medizinische Vorrichtung gemäß einem der Ansprüche 1 bis 6, wobei ein durch die folgende Gleichung (III) dargestelltes Trockenelastizitätsmodul-Verhältnis in einem Bereich von 0,1 oder mehr und 10 oder weniger liegt.

Trockenelastizitätsmodul-Verhältnis = Zugelastizitätsmodul in trockenem Zustand/Zugelastizitätsmodul in hydratisiertem Zustand (III)

**8.** Medizinische Vorrichtung gemäß Anspruch 7, wobei der Zugelastizitätsmodul in trockenem Zustand in einem Bereich von 0,1 MPa oder mehr und 3,5 MPa oder weniger liegt.

9. Medizinische Vorrichtung gemäß einem der Ansprüche 1 bis 8, wobei das Substrat eine Einheit umfasst, die aus der folgenden allgemeinen Formel (1) abgeleitet ist:

[Chemische Formel 1]

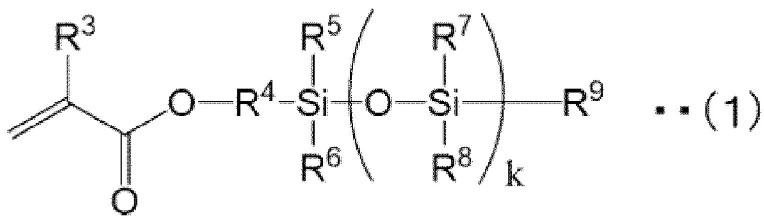

wobei in der allgemeinen Formel (1) $R^3$ ein Wasserstoffatom oder eine Methylgruppe darstellt; wobei $R^4$ eine zweiwertige organische Gruppe darstellt, welche 1 bis 20 Kohlenstoffatome aufweist; wobei $R^5$ bis $R^8$ jeweils unabhängig voneinander eine Alkylgruppe mit 1 bis 20 Kohlenstoffatomen oder eine Arylgruppe mit 6 bis 20 Kohlenstoffatomen darstellen; wobei $R^9$ eine gegebenenfalls substituierte Alkylgruppe mit 1 bis 20 Kohlenstoffatomen oder eine gegebenenfalls substituierte Arylgruppe mit 6 bis 20 Kohlenstoffatomen darstellt; und wobei k eine ganze Zahl in einem Bereich von 1 bis 200 darstellt.

10. Medizinische Vorrichtung gemäß einem der Ansprüche 1 bis 9, wobei das Carbonsäuregruppe-enthaltende Polymer eine oder mehrere Arten ausgewählt aus der Gruppe bestehend aus Polymethacrylsäure, Polyacrylsäure, Polyvinylbenzoesäure, Poly(thiophen-3-essigsäure), Polymaleinsäure, Poly(meth)acryloyloxyethylbernsteinsäure, Poly(meth)acryloyloxyethylhexahydrophtalsäure, Poly(meth)acryloyloxyethylphthalsäure, Poly(meth)acryloyloxyethyl-2-hydroxyethylphthalsäure, Polynorbornencarbonsäure, Poly(meth)acryloyloxyethylsuccinat, und deren Salze, darstellt.

11. Medizinische Vorrichtung gemäß einem der Ansprüche 1 bis 10, wobei das Copolymer mit einer Amidstruktur eine oder mehrere Arten ausgewählt aus der Gruppe bestehend aus einem (Meth)acrylsäure/N-Vinylpyrrolidon-Copolymer, einem (Meth)acrylsäure/N,N-Dimethylacrylamid-Copolymer, einem 2-Acrylamid-2-methylpropansulfonsäure/*N*-Vinylpyrrolidon-Copolymer, und einem 2-Acrylamid-2-methylpropansulfonsäure/*N,N*-Dimethylacrylamid-Copolymer darstellt.

12. Medizinische Vorrichtung gemäß einem der Ansprüche 1 bis 11, wobei das Substrat ein gehärtetes Produkt einer Zusammensetzung zur Bildung eines Substrats ist, wobei
die Zusammensetzung zur Bildung eines Substrats eine polymerisierbare Verbindung mit einer 2-Alkoxyethylgruppe, einen Acylphosphinoxid-basierten Polymerisationsinitiator und einen intramolekularen Wasserstoffabstraktionstyp-Polymerisationsinitiator enthält, und den Acylphosphinoxid-basierten Polymerisationsinitiator in einem Bereich von 0,05 Massen-% bis 0,4 Massen-% und den intramolekularen Wasserstoffabstraktionstyp-Polymerisationsinitiator in einem Bereich von 0,2 Massen-% bis 20 Massen-% enthält.

13. Medizinische Vorrichtung gemäß Anspruch 12, wobei der intramolekulare Wasserstoffabstraktionstyp-Polymerisationsinitiator Methylbenzoylformiat ist.

14. Medizinische Vorrichtung gemäß einem der Ansprüche 1 bis 13, welche eine ophthalmische Linse, ein Hautabdeckmaterial, ein Wundverbandmaterial, ein Hautschutzmaterial, ein Haut-Medikamententräger, ein Infusionsschlauch, ein Gaszufuhrschlauch, ein Drainageschlauch, ein Blutkreislauf, ein Abdeckschlauch, ein Katheter, ein Stent, eine Hülle, ein Schlauchverbinder, ein Zugangsanschluss oder ein endoskopisches Verbandmaterial ist.

15. Verfahren zur Herstellung der medizinischen Vorrichtung gemäß einem der Ansprüche 1 bis 14, umfassend:

einen Schritt des Anordnens des Substrats in einer Lösung, die eine Art des Carbonsäuregruppe-enthaltenden Polymers enthält, und Erwärmen bei 50 °C oder höher, und
einen Schritt des Anordnens des Substrats, das dem Schritt des Erwärmens bei 50 °C oder höher unterzogen wurde, in einer Lösung, die ein Copolymer mit einer Amidstruktur enthält, dessen anfänglicher pH-Wert in einem Bereich von 2 bis 6 eingestellt ist, und Erwärmen bei 60 °C oder höher.

## Revendications

1. Dispositif médical comprenant un substrat et une couche polymère, où la couche polymère est fournie sur au moins une partie d'une surface du substrat et satisfait les exigences suivantes:

   (a) une teneur en eau du dispositif médical dans un état hydraté est dans une plage de 28 % en masse ou plus et 50 % en masse ou moins;
   (b) le substrat a un groupe 2-alcoxyéthyle; et
   (c) la couche polymère contient un type de polymère contenant un groupe acide carboxylique et un type de copolymère ayant une structure amide.

2. Dispositif médical selon la revendication 1, où le groupe 2-alcoxyéthyle est un groupe 2-méthoxyéthyle.

3. Dispositif médical selon la revendication 1, où le groupe 2-alcoxyéthyle est un groupe 2-éthoxyéthyle.

4. Dispositif médical selon l'une quelconque des revendications 1 à 3, où le polymère contenant un groupe acide carboxylique a un poids moléculaire moyen en poids dans une plage de 10 000 ou plus et 500 000 ou moins.

5. Dispositif médical selon l'une quelconque des revendications 1 à 4, où le substrat a le groupe 2-alcoxyéthyle dans une plage de 5 % en masse ou plus et 25 % en masse ou moins.

6. Dispositif médical selon l'une quelconque des revendications 1 à 5, où une demi-vie à l'état humide dérivée de l'équation (I) et de l'équation (II) suivantes est dans une plage de 160 secondes ou plus et 5 000 secondes ou moins:

$$\mathrm{Ln}(F) = -At + B \quad (I)$$

$$\text{demi-vie à l'état humide} = \mathrm{Ln}(2/A) \quad (II),$$

   où F représente une valeur à l'état humide (facteur de fluidité) obtenue par la méthode MS-DWS, et t représente le temps (secondes);
   où l'équation (I) est une approximation linéaire d'une relation entre le logarithme de la valeur à l'état humide F et le temps t; où A représente une pente d'une ligne approximativement droite de l'équation (I), et B représente la section d'axe.

7. Dispositif médical selon l'une quelconque des revendications 1 à 6, où un rapport de module d'élasticité à sec représenté par l'équation (III) suivante est dans une plage de 0,1 ou plus et 10 ou moins.

   Rapport de module d'élasticité à sec = module d'élasticité en traction à l'état sec/module d'élasticité en traction à l'état hydraté (III).

8. Dispositif médical selon la revendication 7, où le module d'élasticité en traction à l'état sec est dans une plage de 0,1 MPa ou plus et 3,5 MPa ou moins.

9. Dispositif médical selon l'une quelconque des revendications 1 à 8, où le substrat comprend une unité dérivée de la formule générale (1) suivante:

[Formule chimique 1]

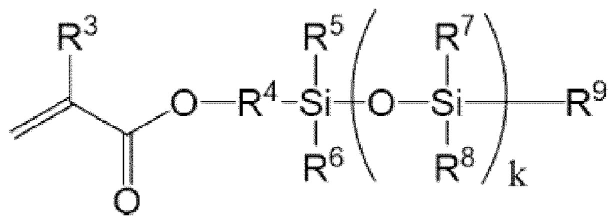

· · (1)

où dans la formule générale (1), $R^3$ représente un atome d'hydrogène ou un groupe méthyle; où $R^4$ représente un groupe organique divalent ayant 1 à 20 atomes de carbone; où $R^5$ à $R^8$ représentent chacun indépendamment un groupe alkyle ayant 1 à 20 atomes de carbone ou un groupe aryle ayant 6 à 20 atomes de carbone; où $R^9$ représente un groupe alkyle éventuellement substitué ayant 1 à 20 atomes de carbone ou un groupe aryle éventuellement substitué ayant 6 à 20 atomes de carbone; et où k représente un nombre entier dans une plage de 1 à 200.

**10.** Dispositif médical selon l'une quelconque des revendications 1 à 9, où le polymère contenant un groupe acide carboxylique représente un ou plusieurs types choisis dans le groupe consistant en l'acide polyméthacrylique, l'acide polyacrylique, l'acide polyvinylbenzoïque, l'acide poly(thiophène-3-acétique), l'acide polymaléique, l'acide poly(méth)acryloyloxyéthylsuccinique, l'acide poly(méth)acryloyloxyéthylhexahydrophtalique, l'acide poly(méth)acryloyloxyéthylphtalique, l'acide poly(méth)acryloyloxyéthyl-2-hydroxyéthylphtalique, l'acide polynorbornènecarboxylique, le succinate de poly(méth)acryloyloxyéthyle, et leurs sels.

**11.** Dispositif médical selon l'une quelconque des revendications 1 à 10, où le copolymère ayant une structure amide représente un ou plusieurs types choisis dans le groupe consistant en un copolymère d'acide (méth)acrylique/N-vinylpyrrolidone, un copolymère d'acide (méth)acrylique/N,N-diméthylacrylamide, un copolymère d'acide 2-acrylamide-2-méthylpropanesulfonique/N-vinylpyrrolidone, et un copolymère d'acide 2-acrylamide-2-méthylpropanesulfonique/*N,N*-diméthylacrylamide.

**12.** Dispositif médical selon l'une quelconque des revendications 1 à 11, où le substrat est un produit durci d'une composition pour former un substrat, où
la composition pour former un substrat comprend un composé polymérisable ayant un groupe 2-alcoxyéthyle, un initiateur de polymérisation à base d'oxyde d'acylphosphine, et un initiateur de polymérisation de type à abstraction d'hydrogène intramoléculaire, et contient l'initiateur de polymérisation à base d'oxyde d'acylphosphine dans une plage de 0,05 % en masse à 0,4 % en masse et l'initiateur de polymérisation de type à abstraction d'hydrogène intramoléculaire dans une plage de 0,2 % en masse à 20 % en masse.

**13.** Dispositif médical selon la revendication 12, où l'initiateur de polymérisation de type à abstraction d'hydrogène intramoléculaire est le formiate de méthylbenzoyle.

**14.** Dispositif médical selon l'une quelconque des revendications 1 à 13, qui est une lentille ophtalmique, un matériau de recouvrement de la peau, un matériau de pansement pour plaie, un matériau de protection de la peau, un support de médicament pour la peau, un tube de perfusion, un tube d'alimentation en gaz, un tube de drainage, un circuit sanguin, un tube de recouvrement, un cathéter, un stent, une gaine, un connecteur de tube, un connecteur d'accès ou un pansement endoscopique.

**15.** Procédé de fabrication du dispositif médical selon l'une quelconque des revendications 1 à 14, comprenant:

une étape de disposition du substrat dans une solution contenant un type du polymère contenant un groupe acide carboxylique et de chauffage à 50°C ou plus, et
une étape de disposition du substrat soumis à l'étape de chauffage à 50°C ou plus dans une solution contenant un copolymère ayant une structure amide dont le pH initial est ajusté dans une plage de 2 à 6 et de chauffage à 60°C ou plus.

Fig. 1

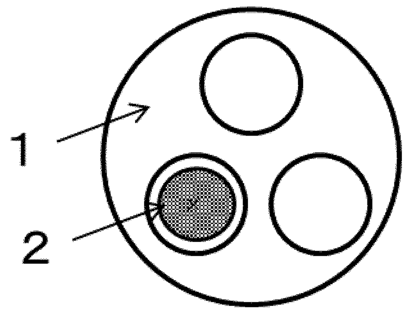


Fig. 2

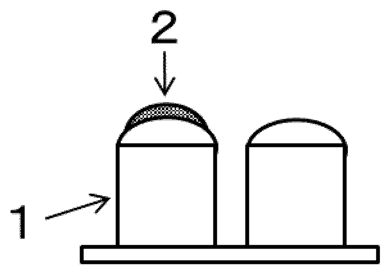


Fig. 3

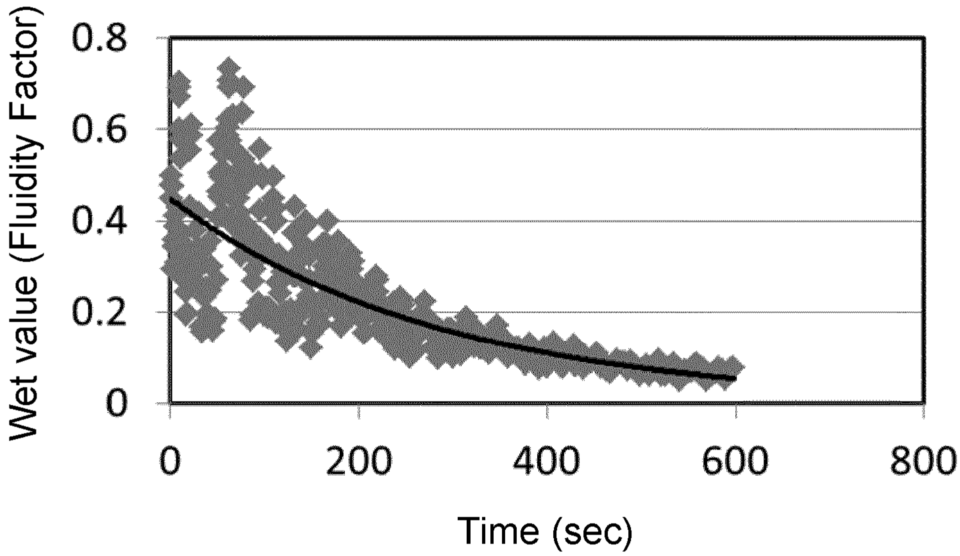

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017169873 A1
- JP 2017023374 A
- JP 2005538767 W
- JP 2014533381 W